# EUROPEAN PATENT APPLICATION

(11) **EP 3 778 586 A1**
(43) Date of publication of application: **17.02.2021**
(21) Application number: 19785966.3
(22) Date of filing: 12.04.2019
(51) Int. Cl.: C07D 401/12, C07F 9/6558, C07D 403/12, C07D 239/94, C07D 401/14, A61K 31/517, A61K 31/5377, A61K 31/675, A61P 35/00

(54) **QUINAZOLINE COMPOUND SERVING AS EGFR TRIPLE MUTATION INHIBITOR AND APPLICATIONS THEREOF**

(30) Priority: 13.04.2018 CN 201810332709
(71) Applicant: East China University of Science and Technology, Shanghai 200237 (CN)
(72) Inventor: LI, Honglin, Shanghai 200237 (CN); DING, Jian, Shanghai 201203 (CN); XU, Yufang, Shanghai 200237 (CN); XIE, Hua, Shanghai 201203 (CN); LI, Qiannan, Shanghai 200237 (CN); ZHANG, Tao, Shanghai 201203 (CN); ZHAO, Zhenjiang, Shanghai 200237 (CN)
(74) Representative: reuteler & cie SA
(86) International application number: PCT/CN2019/082498
(87) International publication number: WO 2019/196938

(57) **Abstract**

Related to a quinazoline compound serving as an EFGR triple mutation inhibitor and applications thereof. Specifically, disclosed are a compound as represented by the following formula (I), a pharmaceutical composition comprising the compound of formula (I), and applications of the compound in treating a related disease mediated by EGFR and in a medicament for treating the related disease mediated by EGFR.

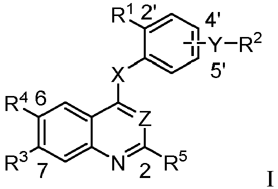

## Description

### Technical field

The present invention belongs to the field of medicine and drug synthesis. In aprticular, the present invention relates to a quinazoline compound as an EGFR triple mutation inhibitor and uses thereof.

### Background

Epidermal growth factor receptor (EGFR, also known as HER-1 or c-erbB-1) is a 170-kDa transmembrane glycoprotein composed of 1186 amino acids. EGFR is composed of three parts: extracellular receptor region; transmembrane region; intracellular tyrosine kinase region. EGFR is a member of the c-erbB family of receptor tyrosine kinases and forms the c-erbB family together with HER2 (c-erbB-2), HER3 (c-erbB-3) and HER4 (c-erbB-4) . The ligands that have been confirmed to bind to EGFR are: epidermal growth factor (EGF), transforming growth factor α(TGFα), two-way regulator, heparin-binding EGF, cytokine, etc. In human tissues, EGF and TGFα are considered to be the two most important ligands of EGFR [2]. When the ligand binds to the extracellular binding domain of EGFR, the ErbB family members will form homodimers or heterodimers, causing the conformational changes of the protein in the cytoplasmic tyrosine kinase region, binding to ATP for autophosphorylation, and then activating downstream signaling molecules (Ras-Raf-MEK/MAPK, Ras-Raf-mitogen-activated protein kinase pathway; PI3K/Akt, phosphatidylinositol 3-kinase/Akt pathway), thereby play a role in maintaining cell growth and proliferation, cell movement, and angiogenesis , inhibition of apoptosis and many other physiological functions.

Due to the key role of EGFR in controlling cell proliferation, survival, and metabolism, interference with its activity can block signal transduction, thereby making EGFR a compelling tumor-targeted therapeutic molecular target, and a drug targeting EGFR has also become a hot spot for tumor treatment. At present, tumor molecular targeted drugs for EGFR are mainly divided into two categories according to their properties: one is monoclonal antibodies that directly act on the extracellular receptor region; the other is small molecule inhibitor that interferes with the intracellular EGFR tyrosine kinase activitys. Monoclonal antibody drugs interact with the extra-membrane ligand binding domain of EGFR, so that endogenous ligands such as EGF cannot bind to EGFR, thereby preventing the signal from entering cells; while small molecule drugs bind to the intracellular tyrosine kinase catalytic region, and inhibit its catalytic activity, thereby blocking cell proliferation signals.

EGFR mutations are mainly concentrated on exons 18-21, which are responsible for encoding the EGFR tyrosine kinase domain. The deletion of exon 19 accounts for 44% of EGFR tyrosine kinase sensitive mutations. The point mutation in exon 21, L858R mutation, accounts for 41% of EGFR tyrosine kinase sensitive mutations. The mutation of residue 719 from glycine to serine, alanine or cysteine accounts for 10% of the total mutations, while insertion or replication mutations in exon 20 accounts for the remaining 5%. The deletion of exon 19 and the L858R point mutation are the most common sensitive mutations. These mutations will enhance the activity of EGFR kinase, thereby improving downstream signaling pathways. Moreover, it is reported that T790M point mutation in exon 20 was found in 50% of patients with drug resistance caused by treatment with EGFR tyrosine kinase inhibitors. This mutation is believed to occur during treatment since it has not been detected in untreated patients. A series of small molecule inhibitors have been derived from these different mutations.

The first generation of EGFR small molecule inhibitors is, such as Gefitinib and Erlotinib. These inhibitors mainly focuse on sensitive mutations, however, with the discovery of T790M resistance mutations, patients gradually develop resistance. Therefore, the second and third generations of EGFR inhibitors were developed, which mainly increase the inhibitory activity by covalently binding the Michael receptor on the molecule to the cysteine 797 residue of the protein.

Although it is promising to use the third-generation of EGFR inhibitors to treat non-small cell lung cancer patients with T790M mutations, drug resistance is gradually emerging. After research, it was found that the occurrence of drug resistance was mainly due to the mutation of Cys797 residue to Ser797 residue, which caused the third-generation of inhibitors to be unable to covalently bind to protein kinases.

Therefore, there is an urgent need to develop a new generation of inhibitors to overcome the EGFR L858R/T790M/C797S triple mutation.

### Summary of the invention

The purpose of the present invention is to provide a class of compounds with novel structures that can be used as ECFR inhibitors.

In the first aspect of the present invention, a compound of Formula I or a stereoisomer or optical isomer, or pharmaceutically acceptable salt thereof is provided: 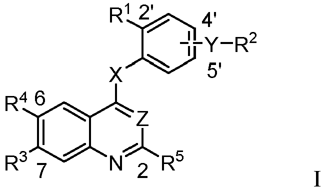 Wherein,
X is a CH₂, NH, O or S;
Z is N or a CH;
Y is absent, -O-, -NHCO-, -CONH-, -NHSO-, -SONH-, -NHCONH- or -NHSONH-;
R1 is a hydrogen, halogen (fluorine, chlorine, bromine, iodine), C1-4 alkyl, halogenated C1-4 alkyl, C1-4 alkoxy, halogenated C1-4 alkoxy, C1-4 alkylthio group, (C1-4 alkyl) (C1-4 alkyl)P(=O)-, nitro or amino;
R2 is selected from the following group: a hydrogen, substituted or unsubstituted phenyl, substituted or unsubstituted phenyl C1-4 alkyl, substituted or unsubstituted benzo C4-7 cycloalkyl, substituted or unsubstituted C4 -7 cycloalkyl C1-4 alkyl, 5 or 6-membered heterocyclic ring containing N or O, and the "substituted" means that one or more hydrogen atoms in the above group are replaced by a group selected from the following group: a halogen, C1-4 alkyl, phenyl,

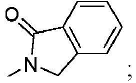

R3 and R4 are each independently selected from the group consisting of a hydrogen, substituted or unsubstituted C1-6 alkyl, substituted or unsubstituted C1-6 alkoxy, substituted or unsubstituted piperazinyl C1-6 alkoxy, substituted or unsubstituted piperidinyl C1-6 alkoxy, substituted or unsubstituted morpholinyl C1-6 alkoxy, or R3 and R4 form -O-C1-6 alkyl-O-; and the "substituted" means that one or more hydrogen atoms in the above-mentioned groups are substituted by a group selected from the group consisting of a halogen, C1-4 alkyl, C1-4 alkoxy, and phenyl;
R5 is a hydrogen, halogen, hydroxyl or amino.

In another preferred embodiment, R2 is selected from the following group:

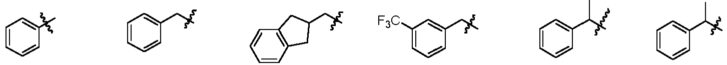

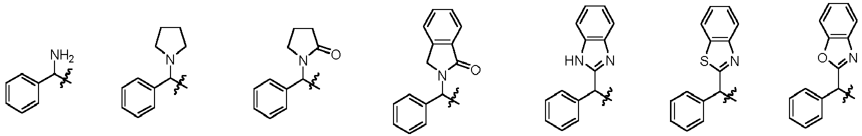

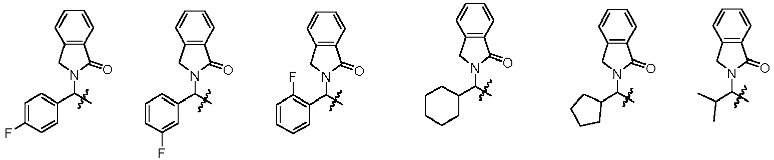

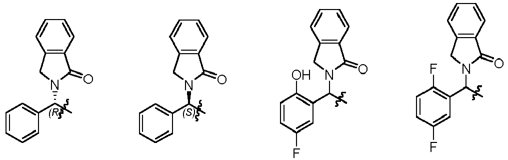

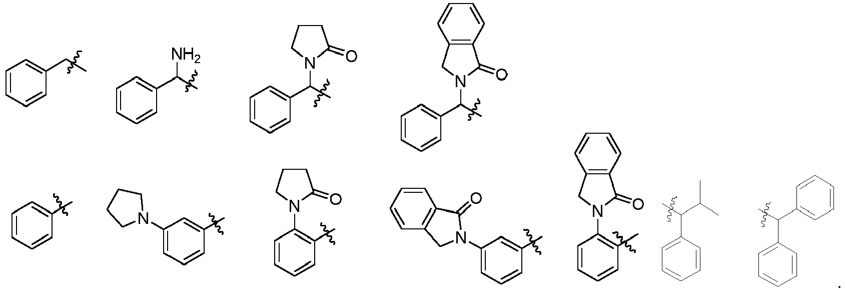

In another preferred embodiment, the compound is represented by Formula II: 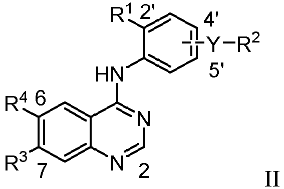 wherein R1, R2, R3, R4, and Y are as defined above.

In another preferred embodiment, the compound is represented by Formula III: 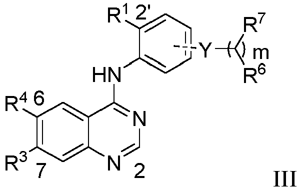
wherein R3, R4, R1 and Y are as described in claim 1;
R6 and R7 are each independently selected from the following group:

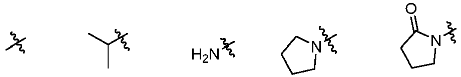

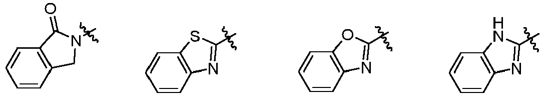

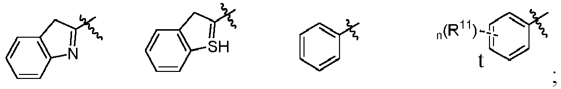

wherein R11 is selected from the group consisting of a hydrogen, halogen, and hydroxyl;
m is 0, 1 or 2;
t is 0, 1, or 2.

In another preferred embodiment, one of R6 and R7 is selected from the following group: 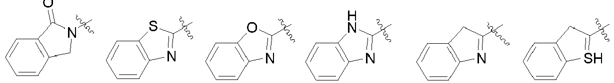 (preferably, 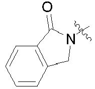 ); and the other is selected from the following group: 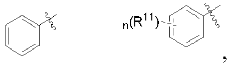 wherein n and R11 are as defined above.

In another preferred embodiment, R11 is selected from the following group: a hydrogen, halogen (preferably, F).

In another preferred embodiment, when Y is -O-, R2 is phenyl or phenyl C1-4 alkyl; preferably, phenyl.

In another preferred embodiment, when Y is -NHCO- or -CONH-, R2 is 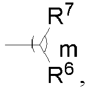 wherein m = 1; R6 and R7 are as defined above.

In another preferred embodiment, R3 and R4 are each independently selected from the following: 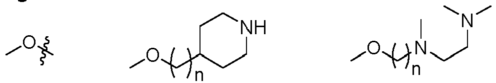 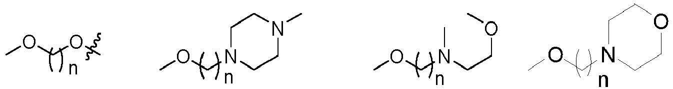 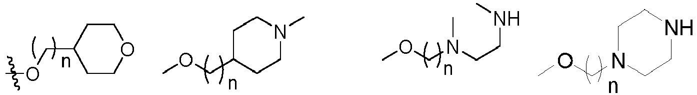 n is 1, 2 or 3.

In another preferred embodiment, the compound is represented by Formula IV: 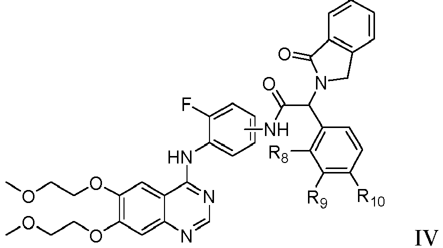 wherein R8, R9, and R10 are each independently selected from the following group: a hydrogen, halogen, and hydroxyl.

In another preferred embodiment, R8 is a hydroxyl or fluorine.

In another preferred embodiment, R9 is a hydrogen.

In another preferred embodiment, R10 is a fluorine.

In another preferred embodiment, the compound is selected from the following group:

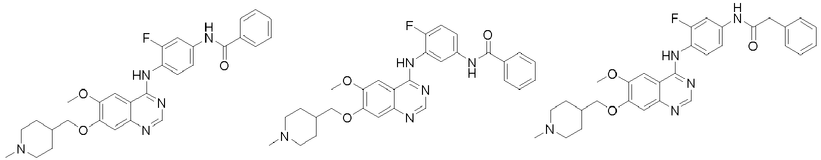

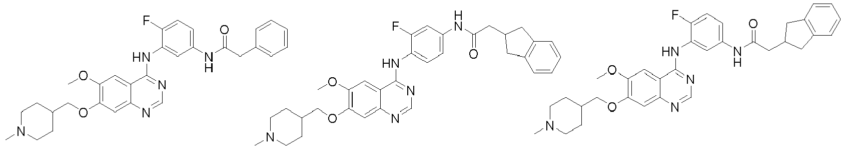

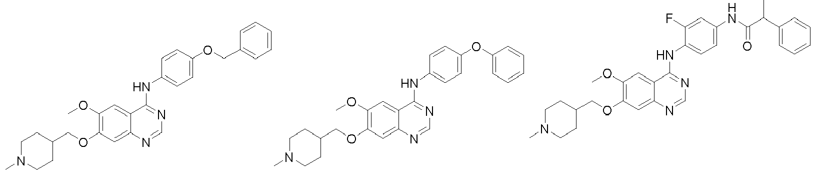

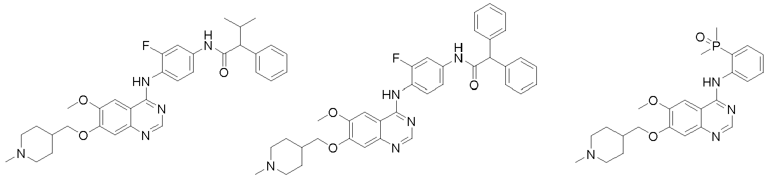

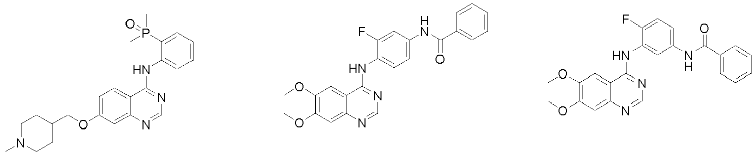

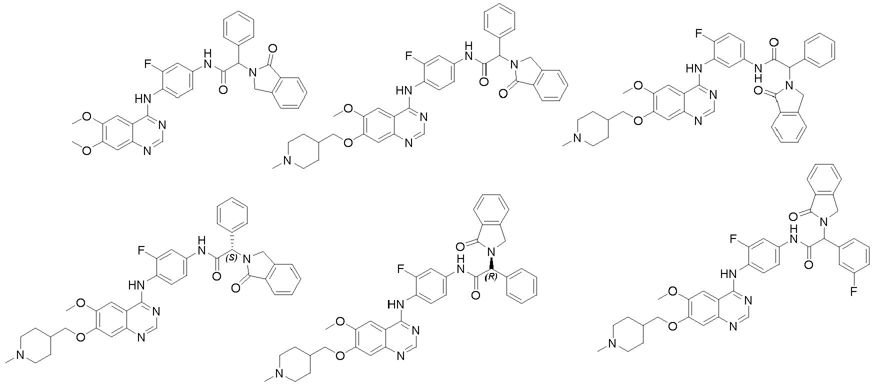

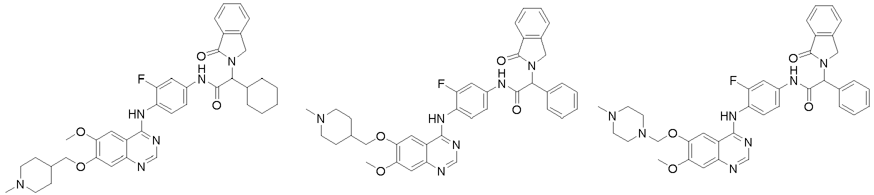

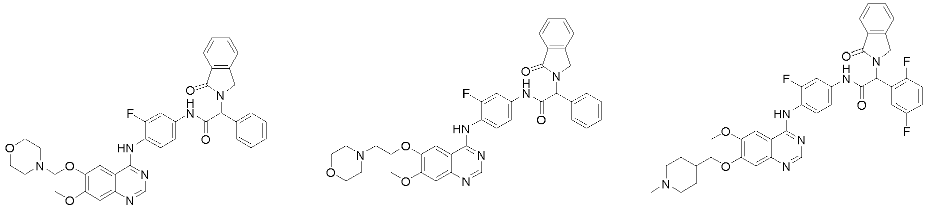

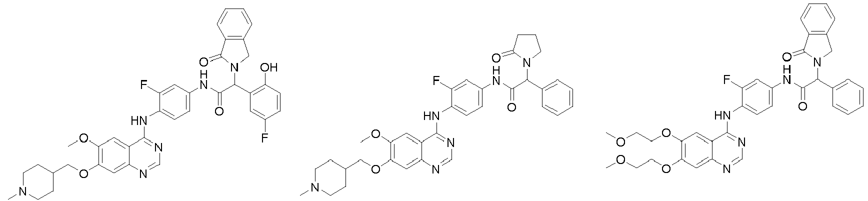

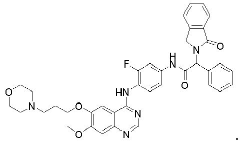

In the second aspect of the present invention, a pharmaceutical composition is provided, comprising the compound described in the first aspect or a stereoisomer or optical isomer, or pharmaceutically acceptable salt thereof, and a pharmaceuitically acceptable carrier or excipient.

In the third aspect of the present invention, the use of the compound described in the first aspect of the present invention or a stereoisomer or optical isomer, or pharmaceutically acceptable salt thereof is provided for preparing a medicament for treating or preventing EGFR-mediated diseases or inhibiting EGFR.

In another preferred embodiment, the EGFR-mediated disease is cancer.

In another preferred embodiment, the cancer is selected from the group consisting of non-small cell lung cancer, small cell lung cancer, lung adenocarcinoma, lung squamous cell carcinoma, breast cancer, prostate cancer, glioma, ovarian cancer, and head Squamous cell carcinoma, cervical cancer, esophageal cancer, liver cancer, kidney cancer, pancreatic cancer, colon cancer, skin cancer, leukemia, lymphoma, gastric cancer, multiple bone marrow cancer and solid tumors.

The present invention provides a treatment method, comprising the step of administering the compound described in the first aspect of the present invention or a stereoisomer or optical isomer, or pharmaceutically acceptable salt thereof to a subject in need thereof.

In another preferred embodiment, the subject in need thereof suffers from an EGFR-mediated disease.

It should be understood that within the scope of the present invention, the above-mentioned technical features of the present invention and the technical features specifically described in the following (such as the embodiments) can be combined with each other to form new or preferred technical solutions, and it is not necessary to repeat them one-by-one.

### Embodiments for carrying out the invention

Through extensive and in-depth research, the inventors unexpectedly discovered a class of quinazoline compounds with excellent EGFR inhibitory activity, based on which the present invention is completed.

### Definition on Terms

Some groups involved herein are defined as follows:
As used herein, "alkyl" refers to a saturated branched or straight chain alkyl with a carbon chain length of 1-10 carbon atoms. The preferred alkyls include alkyls with 1-6, 1-4 or 1-3 carbons in length. Examples of alkyl groups include, but are not limited to, methyl, ethyl, n-propyl, isopropyl, n-butyl, isobutyl, heptyl and the like. An alkyl may be substituted by one or more substituents, for example by a halogen or haloalkyl. For example, an alkyl may be an alkyl group substituted with 1 to 4 fluorine atoms, or the alkyl may be an alkyl group substituted with a fluoroalkyl.

As used herein, "alkoxy" refers to an oxy group substituted by an alkyl group. The preferred alkoxy is an alkoxy having 1 to 6 carbon atoms in length, more preferably an alkoxy having 1 to 4 carbon atoms in length. Examples of alkoxy include, but are not limited to, a methoxy, ethoxy, propoxy and the like. In a specific embodiment, an alkoxy may be a substituted alkoxy, for example, an alkoxy-substituted alkoxy. In a specific embodiment, a C1-C3 alkoxy substituted C1-C3 alkoxy is preferred.

As used herein, "cycloalkyl" refers to a saturated cyclic alkyl containing 3-10, preferably 4-7 ring carbon atoms. Examples of cycloalkyl include, but are not limited to, cyclopropyl, cyclobutyl, cyclohexyl, cycloheptyl, and the like. A cycloalkyl may be substituted by one or more substituents, such as a halogen or haloalkyl. For example, a cycloalkyl groups can be substituted with 1-4 fluorine atoms. In a preferred embodiment, the cycloalkyl group in the present invention is a cyclohexyl.

As used herein, "halogen" refers to a fluorine, chlorine, bromine or iodine.

As used herein, "aryl" refers to a monocyclic, bicyclic or tricyclic aromatic group containing 6 to 14 carbon atoms, including phenyl, naphthyl, phenanthryl, anthryl, indenyl, fluorenyl, tetrahydronaphthyl and indanyl, etc. An aryl may be optionally substituted with 1-5 (for example, 1, 2, 3, 4, or 5) substituents selected from the group consisting of a halogen, C1-4 aldehyde group, C1-6 alkyl, cyano, nitro, amino, hydroxy, hydroxymethyl, halogen-substituted alkyl (e.g. trifluoromethyl), halogen-substituted alkoxy (e.g. trifluoromethoxy), carboxyl, C1-4 alkoxy, ethoxy formyl, N(CH3) and C1-4 acyl, heterocyclyl or heteroaryl, etc.

As used herein, "heterocyclyl" includes, but is not limited to, a 5- or 6-membered heterocyclic group containing 1-3 heteroatoms selected from O, S or N, including but not limited to furyl, thienyl, pyrrolyl, pyrrolidinyl, pyrazolyl, imidazolyl, triazolyl, oxazolyl, pyranyl, pyridyl, pyrimidinyl, pyrazinyl, piperidinyl, morpholinyl, etc.

As used herein, "optionally substituted" means that the group modified by this term can be optionally substituted by 1-5 (for example, 1, 2, 3, 4, or 5) substituents selected from the following group: a halogen, C1-4 aldehyde group, C1-6 linear or branched alkyl, cyano, nitro, amino, hydroxyl, hydroxymethyl, halogen-substituted alkyl (e.g. trifluoromethyl), halogen-substituted alkoxy (for example, trifluoromethoxy), carboxyl, C1-4 alkoxy, ethoxyformyl, N(CH3) and C1-4 acyl.

### Active ingredient

A series of quinoline compounds with novel structures, as shown in general formula I is provided, and structurally characterized in the present invention.

The compound of the present invention may also be a stereoisomer or optical isomer, or a pharmaceutically acceptable salt thereof of the compound represented by formula I.

Examples of the pharmaceutically acceptable salt of the compound of the present invention include, but are not limited to, a salt formed by the compound of the present invention and an acid. The acid suitable for forming a salt includes but not limited to: an inorganic acid, such as hydrochloric acid, hydrobromic acid, hydrofluoric acid, sulfuric acid, nitric acid, phosphoric acid, an organic acid, such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and an acidic amino acid, such as aspartic acid and glutamic acid.

Unless otherwise specified, the structural formula described in the present invention is intended to include all isomeric forms (such as enantiomers, diastereomers and geometric isomers (or conformational isomers)): for example, R and S configurations containing an asymmetry center, (Z) and (E) isomers of the double bond, etc. Therefore, a single stereochemical isomer of the compound of the present invention or a mixture of enantiomers, diastereomers or geometric isomers (or conformational isomers) thereof will fall within to the scope of the present invention.

### Pharmaceutical composition

The compound of the present invention exhibits excellent EGFR kinase-inhibiting activities (especially EGFR triple mutation-inhibiting activities), therefore, the compound of the present invention and various crystal forms, pharmaceutically acceptable inorganic or organic salts, hydrates or solvates thereof, and the pharmaceutical composition containing the compound of the present invention as the main active ingredient can be used to prevent and/or treat (stabilize, alleviate or cure) EGFR kinase related diseases.

The pharmaceutical composition of the present invention comprises a safe and effective amount of the compound of the present invention and a pharmaceutically acceptable excipient or carrier. The "safe and effective amount" means that the amount of the compound is sufficient to significantly improve the condition without causing serious side effects. Generally, the pharmaceutical composition contains 1-2000 mg of the compound of the present invention per agent, and more preferably, 10-200 mg of the compound of the present invention per agent. Preferably, the "one dose" is one capsule or tablet.

The pharmaceutical composition of the present invention comprises a safe and effective amount of the compound of the present invention and a pharmaceutically acceptable excipient or carrier. The "safe and effective amount" means that the amount of the compound is sufficient to significantly improve the condition without causing serious side effects. Generally, the pharmaceutical composition contains 1-2000 mg of the compound of the present invention per agent, and more preferably, 10-200 mg of the compound of the present invention per agent. Preferably, the "one dose" is one capsule or tablet.

"Pharmaceutically acceptable carrier" refers to: one or more compatible solid or liquid fillers or gel substances, which are suitable for human use, and must have sufficient purity and sufficiently low toxicity. As used herein, "compatibility" means that each component in the composition can be blended with the compound of the present invention without significantly reducing the efficacy of the compound. Examples of pharmaceutically acceptable carriers include cellulose and a derivative thereof (such as sodium carboxymethyl cellulose, sodium ethyl cellulose, cellulose acetate, etc.), gelatin, talc, and solid lubricants (such as stearic acid, Magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween®), wetting agents (such as sodium lauryl sulfate), coloring agents, flavoring agents, stabilizers, antioxidants, preservatives, pyrogen-free water, etc.

The method for administering the compound or pharmaceutical composition of the present invention is not particularly limited, and representative administration methods include (but are not limited to): oral, parenteral (intravenous, intramuscular, or subcutaneous) administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following ingredients: (a) fillers or compatibilizers, for example, Starch, lactose, sucrose, glucose, mannitol and silicic acid; (b) binders such as hydroxymethyl cellulose, alginate, gelatin, polyvinylpyrrolidone, sucrose and gum arabic; (c) humectants, For example, glycerin; (d) disintegrating agents, such as agar, calcium carbonate, potato starch or tapioca starch, alginic acid, certain complex silicates, and sodium carbonate; (e) slow solvents, such as paraffin; (f) Absorption accelerators, such as quaternary amine compounds; (g) wetting agents, such as cetyl alcohol and glyceryl monostearate; (h) adsorbents, such as kaolin; and (i) lubricants, such as talc, hard Calcium fatty acid, magnesium stearate, solid polyethylene glycol, sodium lauryl sulfate, or mixtures thereof. In capsules, tablets and pills, the dosage form may also contain buffering agents.

Solid dosage forms, such as tablets, sugar pills, capsules, pills and granules can be prepared with coatings and shell materials, such as enteric coatings and other materials known in the art. They may contain opacifying agents, and the active compound or the release of the compound in such a composition may be released in a certain part of the digestive tract in a delayed manner. Examples of embedding components that can be used are polymeric substances and waxes. If necessary, the active compound can also be formed into microcapsules with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures thereof.

In addition to these inert diluents, the composition may also contain adjuvants, such as wetting agents, emulsifying and suspending agents, sweetening agents, flavoring agents and perfumes.

In addition to the active compound, the suspension may contain suspending agents, for example, ethoxylated isostearyl alcohol, polyoxyethylene sorbitol and sorbitan esters, microcrystalline cellulose, aluminum methoxide and agar, or mixtures thereof, and the like.

The composition for parenteral injection may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The compound of the present invention can be administered alone or in combination with other pharmaceutically acceptable compounds.

When administered in combination, the pharmaceutical composition also includes one or more (2, 3, 4, or more) other pharmaceutically acceptable compounds. One or more of the other pharmaceutically acceptable compounds can be administered simultaneously, separately or sequentially with the compounds of the invention.

When using the pharmaceutical composition, a safe and effective amount of the compound of the present invention is applied to a mammal (such as a human) in need of the treatment, wherein the administered dosage is a pharmaceutically effective dosage. For a human with a body weight of 60 kg, the daily dosage is usually 1 to 2000 mg, preferably 20 to 500 mg. When determining a specific dosage, factors such as the route of administration, the patient's health status and the like should also be considered, which are within the skill of a skilled physician.

### Preparation method

The preparation method for the compound of the present invention may be a conventional method in the art, or the synthetic route of the present invention may be adopted, 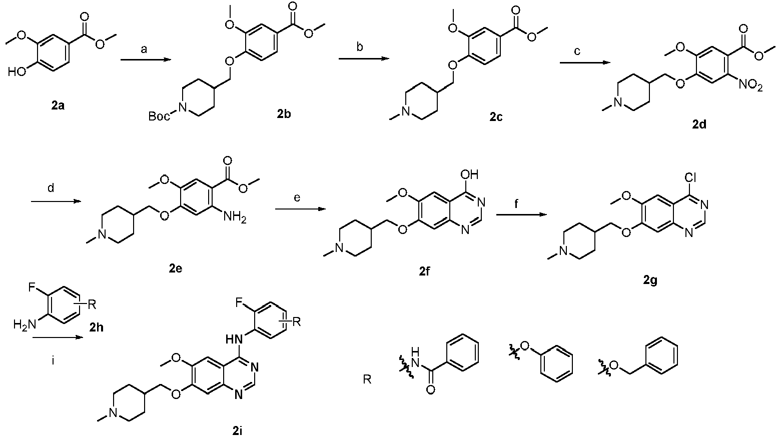 wherein the reagents and conditions of each step are listed as follows:
(a) tert-butyl 4-((tosyloxy)methyl)piperidine-1-carboxylate, DMF, K₂CO₃; 120°C, 5h;
(b) HCHO, HCOOH, 120°C, 8h;
(c) fuming nitric acid, DCM, 25°C, overnight;
(d) Pd/C, MeOH/DCM, H₂, 25°C, 4h;
(e) formamidine acetate, CH₃OCH₂CH₂OH, 120°C, 8h;
(f) SOCl₂, DMF, 80°C, 3h;
(g) THF, Et₃N, 60°C, 5h;
(h) Pd/C, H₂, MeOH/DCM, 25°C, 5h;
(i) i-PrOH, HCl, 80°C, 8h.

Wherein the preparation method for compound 2h is shown as follows:

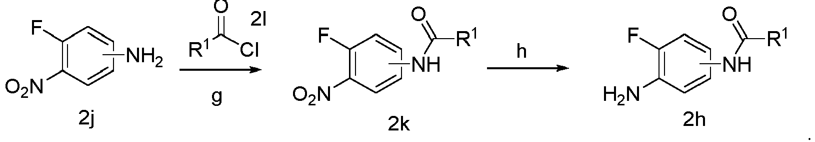

The present invention will be further described below in combination with specific embodiments. It should be understood that these embodiments are only used to illustrate the present invention and not to limit the scope of the present invention. The experimental methods, specific conditions of which are not indicated in the following examples, usually follow the conventional conditions or the conditions recommended by the manufacturer. Unless otherwise specified, percentages and parts are weight percentages and parts by weight.

The experimental materials and reagents used in the following examples are commercially available unless otherwise specified.

### Example 1: Synthesis of Compound 1

### Synthesis of methyl 4-(N-Boc-4-piperidinylmethoxy)-3-methoxybenzoate

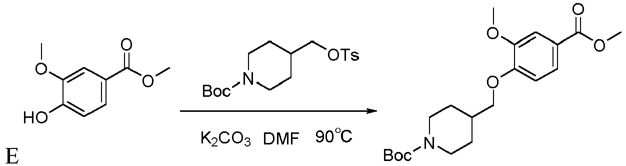

Methyl trioxalate (1g, 5.49mmol) was weighted into a 100 mL eggplant-shaped bottle, and anhydrous potassium carbonate (1.52g, 10.98mmol) and about 50 mL of DMF were added, and stirred for about 15 min at room temperature. Tert-butyl tert-butyl-4-((tosyloxy)methyl)piperidine-1-carboxylate (2.75g, 7.41mmol) was added, heated to 95°C and refluxed for 3h. The reaction was confirmed as being completed by TLC. The reaction mixture was extracted with saturated sodium chloride/ethyl acetate and dried over anhydrous sodium sulfate. Most of the solvent was removed *in vacuo,* and the residual DMF was removed by a diaphragm pump at 70°C for about 20 minutes. The crude product was separated by silica gel-column chromatography (petroleum ether / ethyl acetate = 8: 1) to obtain 2 g of methyl 4-(N-Boc-4-piperidinylmethoxy)-3-methoxybenzoate with a yield of 86%. ¹H NMR(400MHz, DMSO): *δ* 7.57 (d, *J* = 8.4Hz, 1H), 7.45 (s, 1H), 7.07 (d, *J* = 8.4Hz, 1H), 3.97 (d, *J* = 12Hz, 2H), 3.9 (d, *J=* 6.4Hz, 2H), 3.82 (s, 3H), 3.81 (s, 3H), 2.74 (s, 2H), 1.95-1.85 (m, 1H), 1.75 (d, *J* = 8.4Hz, 2H), 1.4 (s, 9H), 1.15 (m, 2H).

### Synthesis of Methyl 4-(N-methyl-4-piperidinylmethoxy)-3-methoxybenzoate

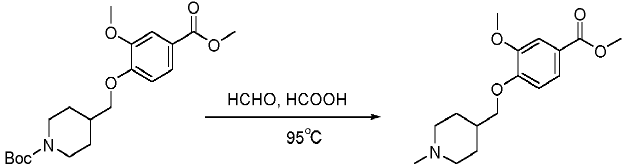

Methyl 4-(N-Boc-4-piperidinylmethoxy)-3-methoxybenzoate (1g, 2.64mmol) was weighted into a 100 mL eggplant-shaped flask, and about 12 mL of formic acid was added, and stirred for about 30 min at room temperature for dissolving it. Afterwards, formaldehyde solution (4 mL, 47.44 mmol, 37%) was slowly added dropwise, heated under reflux at 95°C in an atomsphere of argon for about 6 h. The reaction was confirmed as being completed by TLC. The solvent was removed by a diaphragm pump. The crude product was separated by silica gel column chromatography (dichloromethane / methanol = 30: 1) to obtain 730 mg of methyl
4-(N-methyl-4-piperidinylmethoxy)-3-methoxybenzoate as a white solid with a yield of 94%. ¹H NMR(400MHz, DMSO): *δ* 7.57 (d, *J=* 8.4Hz, 1H), 7.42 (s, 1H), 7.10 (d, *J=* 8.4Hz, 1H), 3.94 (d, *J* = 6Hz, 2H), 3.82 (s, 3H), 3.81 (s, 3H), 3.25 (d, *J* = 12Hz, 2H), 2.70 (dd, *J₁* = 11.2Hz, *J₂* = 22.8Hz, 2H), 2.6 (s, 3H), 1.97-1.80 (m, 1H), 1.88 (d, *J=* 21.6Hz, 2H), 1.49-1.40 (m, 2H).

### Synthesis of methyl 6-nitro-4-(N-methyl-4-piperidinylmethoxy)-3-methoxybenzoate

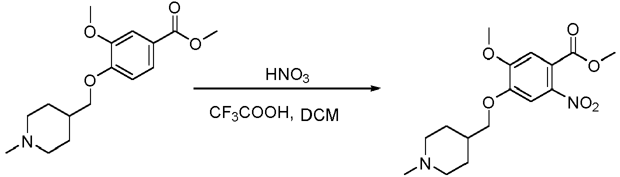

Methyl 4-(N-methyl-4-piperidinylmethoxy)-3-methoxybenzoate (500 mg, 1.7 mmol) was weighted into a 100 mL eggplant-shaped flask, and about 20 mL of DCM was added to dissolve it. About 2 mL of trifluoroacetic acid was slowly added dropwise in an ice bath, and 24 M fuming nitric acid (400 uL, 8.52mmol) was added dropwise within 15 minutes. The reaction was conducted at room temperature for about 6 h. After the reaction was completed, the pH was adjusted to 7 with saturated sodium bicarbonate aqueous solution, and the resulting system was extracted with saturated sodium chloride / ethyl acetate, and dried over anhydrous sodium sulfate. The solvent was removed *in vacuo,* and the crude product was separated by silica gel column chromatography (dichloromethane / methanol = 30: 1) to obtain 450 mg of light yellow oily methyl 6-nitro-4-(N-methyl-4-piperidinylmethoxy)-3-methylbenzoate with a yield of 78%. ¹H NMR(400MHz, DMSO): *δ* 7.62 (s, 1H), 7.31 (s, 1H), 3.97 (d, *J* = 6Hz), 3.92 (s, 3H), 3.83 (s, 3H), 2.77 (d, *J=* 11.2 Hz, 2H), 2.15 (s, 3H), 1.86 (t, *J* = 11.2Hz, 2H), 1.76-1.74 (m, 1H), 1.72 (d, *J* = 10.4Hz, 2H), 1.30-1.25 (m, 2H).

### Synthesis of methyl 6-amino-4-(N-methyl-4-piperidinylmethoxy)-3-methoxybenzoate

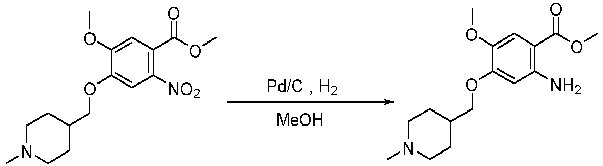

Methyl 6-nitro-4-(N-methyl-4-piperidinylmethoxy)-3-methoxybenzoate (400mg, 1.36mmol) was weighted into a 250 mL eggplant-shaped bottle, about 100 mL of methanol was added to dissolve it, and Pd/C (40 mg, 10%) was added. The reaction was conducted under hydrogen for about 6 hours. The reaction was confirmed as being completed by TLC. Pd/C was removed through diatomaceous earth, and the solvent was removed *in vacuo* to obtain about 350 mg of methyl 6-amino-4-(N-methyl-4-piperidinylmethoxy)-3-methoxybenzoate as an oily liquid with a yield of 96%, which was directly used in the next step.

### Synthesis of 6-methoxy-7-(N-methyl-4-piperidinylmethoxy)-3,4-dihydroquinazolin-4-one

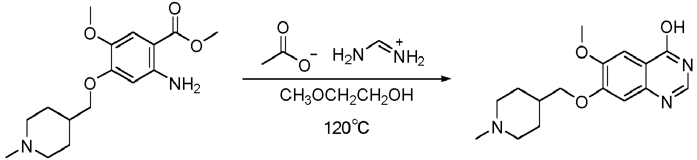

Methyl 6-amino-4-(N-methyl-4-piperidinylmethoxy)-3-methoxybenzote (350 mg, 1.13 mmol) was weighted into a 250 mL eggplant-shaped bottle, about 100 mL of ethylene glycol monomethyl ether was added to dissolve it, and formamidine acetate (355 mg, 3.4 mmol) was added in batches for 40 minutes. The reaction was refluxed at 120°C for about 3 hours. It was found by TLC that the raw materials were not consumed. Formamidine acetate (240 mg, 2.27mmol) was added in two batches (one batch every 1 hour). After about 5 hours, it was found by TLC that a small amount of raw materials were not consumed yet. The reaction was quenched, the solvent was removed *in vacuo,* the pH was adjusted to 9 with saturated sodium bicarbonate aqueous solution, and the solution was extracted with saturated sodium chloride / dichloromethane for several times, dried over anhydrous sodium sulfate, and the solvent was removed *in vacuo.* The crude product was separated by silica gel column chromatography (dichloromethane / methanol = 10: 1) to obtain 6-methoxy-7-(N-methyl-4-piperidinylmethoxy)-3,4-dihydroquinazolin-4-one as a pale yellow solid (100 mg, a yield of 30%). ¹H NMR(400MHz, DMSO): *δ* 7.98 (s, 1H), 7.45 (s, 1H), 7.13 (s, 1H), 3.98 (d, *J=* 6.0 Hz, 2H), 3.87 (s, 3H), 2.92 (d, *J* = 10.8Hz, 2H), 2.29 (s, 3H), 2.13 (t, *J*=10.4Hz, 2H), 1.85-1.81 (m, 1H), 1.79 (d, *J=* 12Hz, 2H), 1.39-1.32 (m, 2H).

### 4-chloro-6-methoxy-7-((1-methylpiperidin-4-yl)methoxy)quinazoline

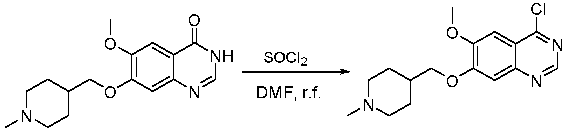

About 5 mL of thionyl chloride was added into a 50 mL eggplant-shaped bottle, and about 1 drop of N,N-dimethylformamide was added, and stirred for about 30 minutes in an ice bath. 100 mg of 6-methoxy-7-(N-methyl -4-piperidinylmethoxy)-3,4-dihydroquinazolin-4-one as a pale yellow solid was placed into the above-mentioned eggplant-shaped bottle, and stirred at room temperature for about 12 h. A small amount of sample was taken and TCL-detected. After the raw materials were consumed, the solvent was removed *in vacuo.* The pH was adjusted to 9 with saturated aqueous sodium bicarbonate solution, and the resulting system was extracted for several times with saturated sodium chloride / dichloromethane, and dried over anhydrous sodium sulfate. The solvent was removed *in vacuo.* The crude product was separated by silica gel column chromatography (dichloromethane / methanol = 10: 1) to obtain
4-chloro-6-methoxy-7-((1-methylpiperidin-4-yl)methoxy)quinazoline as a pale yellow solid (75mg, a yield of 75%). ¹H NMR(400MHz, DMSO): *δ 8.87* (s, 1H), 7.45 (s, 1H), 7.40 (s, 1H), 4.00 (d, *J* = 6.4Hz, 2H), 4.00 (s, 3H), 2.78 (d, *J* = 11.2Hz, 2H), 2.16 (s, 3H), 1.90 (t, *J*=13.2Hz, 2H),1.79 (d, *J* = 12Hz, 3H), 1.35-1.32 (m, 2H).

### N-(3-Fluoro-4-nitrophenyl)-4-methoxybenzamide

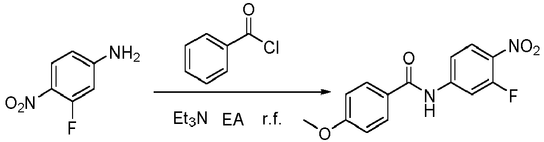

3-fluoro-4-nitroaniline (2 g, 12.55 mmol) was weighted into a 250 mL eggplant-shaped flask, and ethyl acetate was added to dissolve it. Benzenesulfonyl chloride (2 mL, 15.07 mmol) and triethylamine (2.2 mL, 15.07 mmol) were added dropwise. The reactiom mixture was stirred at room temperature under argon for 1 hour, and then refluxed for 6 hours. The raw materials were consumed as confirmed by TLC. The solvent was removed *in vacuo.* The crude product was separated by silica gel column chromatography (dichloromethane / petroleum ether = 3: 1) to obtain 2.78 g of N-(3-fluoro-4-nitrophenyl)-4-methoxybenzamide as a pale yellow solid with a yield of 80%. ¹H NMR(400MHz, DMSO): δ 11.03 (s, 1H), 8.22 (t, *J*= 9.2Hz, 1H), 8.10 (d, *J*= 14.4Hz, 1H), 8.00 (d, *J* = 7.2Hz, 1H), 7.82 (d, *J*= 9.2Hz, 1H), *7.65* (t, *J*= 7.2Hz, 1H), 7.57 (t, *J*= 7.6Hz, 1H).

### N-(4-amino-3-fluorophenyl)benzamide

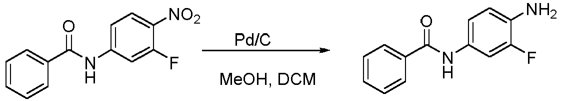

N-(3-fluoro-4-nitrophenyl)-4-methoxybenzamide (500 mg, 1.92 mmol) was weighted into a 250 mL eggplant-shaped flask, and methanol was added to dissolve it. Palladium on carbon (50 mg, 10%) was added. The reaction system was vacuumed and stirred at room temperature for 3 hours under hydrogen. The raw materials were consumed as confirmed by TLC. The Pd/C was removed through diatomaceous earth, and the solvent was removed *in vacuo* to obtain about 430 mg of N-(4-amino-3-fluorophenyl)benzamide with a yield of 97%, which was directly used in the next step.

### N-(3-Fluoro-4-((6-methoxy-7-((1-methylpiperidin-4-yl)methoxy)quinazolin-4-yl)amino)phenyl

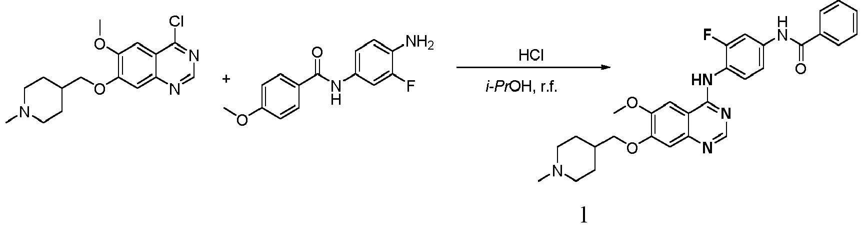

N-(4-amino-3-fluorophenyl)benzamide (180mg, 0.56mmol) was weighted into the above-mentioned eggplant-shaped bottle, and about 15 mL of isopropanol was added to dissolve it. 4-chloro-6-methoxy-7-((1-methylpiperidin-4-yl)methoxy)quinazoline (196 mg, 0.75 mmol) was taken in a test tube, dissolved by adding isopropanol, and then added dropwise to the eggplant-shaped bottle. The reaction was conducted at room temperature for about 1 h, and the reaction solution was slightly reddish. 2 drops of 2 N hydrochloric acid was added, and refluxed at 80°C for about 6 h, so that white precipitate was produced. The raw materials were consumed as detected by TLC. The reaction system was suction-filtered and the solid precipitate was washed with a small amount of isopropanol. The precipitate was dissolved in dichloromethane, the pH was adjusted to 9 with saturated sodium bicarbonate solution, and the resulting solution was extracted for several times with saturated sodium chloride/dichloromethane, and dried over anhydrous sodium sulfate. The solvent was removed *in vacuo.* The crude product was separated by silica gel column chromatography (dichloromethane / methanol = 10: 1) to obtain N-(3-fluoro-4-((6-methoxy-7-((1-methylpiperidine-4-yl)methoxy)quinazolin-4-yl)amino)phenyl) as a white solid (180mg, yield of 62%). ¹H NMR(400MHz, DMSO): δ 10.49 (s, 1H), 9.48 (s, 1H), 8.34 (s, 1H), 7.98 (d, J = 7.2Hz, 2H), 7.87 (d, J = 12.8Hz, 1H), 7.83 (s, 1H), 7.6 (s, 1H), 7.57 (t, J = 7.6Hz, 2H), 7.53 (t, J = 8.8Hz, 1H), 7.6 (s, 1H), 4.99 (d, J = 6Hz, 2H), 3.95 (s, 3H), 2.79 (d, J = 11.2Hz, 2H), 2.17 (s, 3H), 1.89 (t, J = 11.2Hz, 3H), 1.37-1.34 (m, 2H). 13C NMR(100 MHz, DMSO-d6) δ 165.69, 157.37, 153.60, 153.10, 148.94, 148.77, 138.04, 137.94, 134.64, 131.75, 128.43, 127.67, 121.81, 121.68, 115.86, 108.45, 107.61, 102.01, 72.70, 56.12, 54.83, 46.16, 34.57, 28.45. HRMS (ESI) (m/z): [M + H]+ calcd for C₂₉H₃₃N₈O₃, 516.2433; found, 516.2410. HPLC purity: 96.92%, retention time = 11.06 min.

All of the following compounds were synthesized using the same or similar routes as in Example 1.

### Example 2

### N-(4-Fluoro-3-((6-methoxy-7-((1-methylpiperidin-4-yl)methoxy)quinazolin-4-yl)amino)phenyl)ben zamide

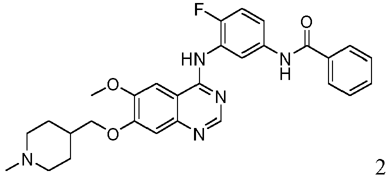

Light yellow solid, the yield of 45%. ¹H NMR (400 MHz, DMSO-d6): δ 10.38 (s, 1H), 9.58 (s, 1H), 8.36 (s, 1H), 8.05 (d, J = 7.2Hz, 1H), 7.87(d, J = 7.2Hz, 2H), 7.84 (s, 1H), 7.67-7.53 (m, 4H), 7.31 (t, J =12Hz, 1H), 7.18 (s, 1H), 4.01(d, J = 6Hz, 2H), 3.95(s, 3H), 2.83 (d, J = 11.2Hz, 2H), 2.21 (s, 3H), 1.96 (t, J = 12Hz, 3H), 1.78(d, J = 10.4Hz, 2H), 1.30-1.58 (m, 2H). 13C NMR(100 MHz, DMSO-d6) δ 170.74, 157.40, 153.54, 153.10, 148.90, 146.73, 142.50, 128.62, 126.13, 124.39, 114.60, 108.43, 107.62, 102.02, 72.58, 56.12, 54.65, 48.55, 45.86, 42.02, 38.30, 36.20, 34.37, 28.22. HRMS (ESI) (m/z): [M + H]+, calcd for C₂₉H₃₃N₈O₃, 516.2433; found, 516.2410. HPLC purity: 95.36%, retention time = 10.78 min.

### Example 3

### N-(3-Fluoro-4-((6-methoxy-7-((1-methylpiperidin-4-yl)methoxy)quinazolin-4-yl)amino)phenyl)-2-p henylacetamide

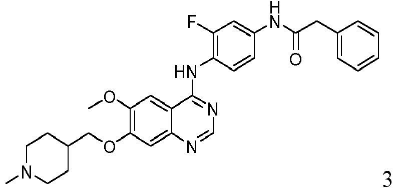

White solid, yield of 40%. ¹H NMR (400 MHz, DMSO-d6): δ 10.42(s, 1H), 10.41(s, 1H), 8.30(s, 1H), 7.80(s, 1H), 7.73(dd, J1=12.4Hz, J2=2Hz, 1H), 7.46 (t, J=8.4Hz, 1H), 7.37-7.32 (m, 5H), 7.26 (m, 1H), 4.00(d, J=6Hz, 2H), 3.96(s, 3H), 3.68(s, 2H), 2.80(d, J=12Hz, 2H), 2.17(s, 3H), 1.89 (t, J=11.2Hz, 2H), 1.78-1.76(m, 3H). 13C NMR(100 MHz, DMSO-d6): δ 169.32, 157.36, 153.58, 153.08, 148.92 146.75, 135.73, 129.10, 128.31, 126.57, 114.67, 108.42, 107.61, 106.75, 102.00, 72.67, 56.11, 54.81, 48.56, 46.11, 43.26, 34.54, 28.42. HRMS (ESI) (m/z): [M + H]+ calcd for C₂₉H₃₃N₈O₃, 530.2567; found, 530.2543. HPLC purity: 95.00 %, retention time = 11.20 min.

### Example 4

### N-(4-Fluoro-3-((6-methoxy-7-((1-methylpiperidin-4-yl)methoxy)quinazolin-4-yl)amino)phenyl)-2-p henylacetamide

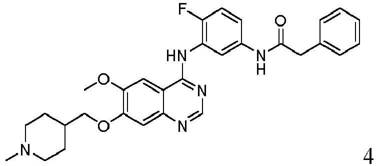

White solid, yield of 43%. ¹H NMR (400 MHz, DMSO-d6): δ 10.29(s, 1H), 9.50(s, 1H), 8.35(s, 1H), 7.87(dd, J1=7.2Hz, J2=2.8Hz, 1H), 7.80(s, 1H), 7.44-7.45 (m, 1H), 7.34-7.24 (m, 6H), 7.17(s, 1H), 4.00(d, J=6Hz, 2H), 3.94(s, 3H), 3.65(s, 2H), 2.80(d, J=12Hz, 2H), 2.17(s, 3H), 1.89 (t, J=11.2Hz, 2H), 1.78-1.76(m, 3H). 13C NMR(100 MHz, DMSO-d6): δ 169.06, 157.03, 153.67, 153.00, 149.00 146.85, 135.90, 129.00, 128.29, 126.52, 118.55, 115.90, 108.51, 107.60, 102.00, 72.71, 56.10, 54.83, 46.16, 43.26, 34.56, 26.44. HRMS (ESI) (m/z): [M + H]+ calcd for C₂₉H₃₃N₈O₃, 530.2567; found, 530.2543. HPLC purity: 97.22%, retention time = 11.05 min.

### Example 5.

### 2-(2,3-Dihydro-1H-inden-2-yl)-N-(3-fluoro-4-((6-methoxy-7-((1-methylpiperidin)-4-yl)methoxy)qui nazolin-4-yl)amino)phenyl)acetamide

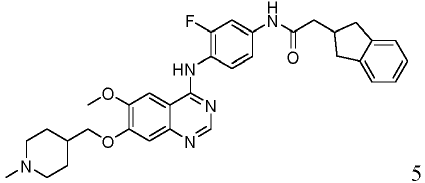

Light yellow solid, yield of 48%. ¹H NMR (400 MHz, DMSO-d6): δ 10.2 (s, 1H), 9.44 (s, 1H), 8.31 (s, 1H), 7.82 (s, 1H), 7.74 (s, 1H), 7.41 (s, 1H), 7.35 (d, J = 8.8Hz, 1H), 7.23 (t, J = 3.2Hz, 2H), 7.18 (s, 1H), 7.14-7.11 (m, 2H), 4.01 (d, J = 6Hz, 2H), 3.94 (s, 3H), 3.17 (s, 2H), 3.13-3.05 (m, 3H), 2.90-2.83 (m, 2H), 2.87 (s, 1H), 2.83 (s, 1H), 2.22 (s, 3H), 1.98 (t, J = 10.8Hz, 2H), 1.78 (d, J = 10.4Hz, 2H), 1.30-1.58 (m, 2H). 13C NMR(100 MHz, DMSO-d6): δ 170.74, 157.40, 153.54, 153.10, 148.90, 146.73, 142.50, 126.13, 124.39, 114.60, 108.43, 107.62, 106.71, 106.46, 102.02, 72.58, 56.12, 54.65, 46.55, 45.86, 42.02, 36.30, 36.20, 34.37, 28.22. HRMS (ESI) (m/z): [M + H]⁺ calcd for C₂₉H₃₃N₈O₃, 570.2880; found, 570.2889. HPLC purity: 96.71%, retention time = 12.41 min.

### Example 6 2-(2,3-Dihydro-1H-inden-2-yl)-N-(4-fluoro-3-((6-methoxy-7-((1-methylpiperidin-4-yl)methoxy)quinazolin-4-yl)amino)phenyl)acetamide

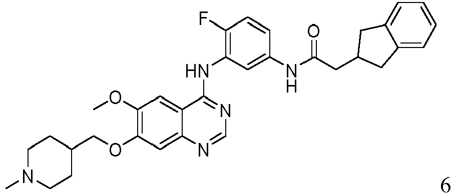

White solid, yield of 32%. ¹H NMR (400 MHz, DMSO-d6): δ 10.2(s, 1H), 9.67(s, 1H), 8.35(s, 1H), 7.90(s, 1H), 7.50(s, 1H), 7.25-7.20 (m, 4H), 7.12-7.10 (m, 2H), 4.03(d, J=6Hz, 2H), 3.96(s, 3H), 3.08-3.02(m, 4H), 2.90-2.82(m, 2H), 2.87(s, 1H), 2.83(s, 1H), 2.42(s, 5H), 2.02-1.86(m, 4H), 1.52-1.48(m, 2H). 13C NMR(100 MHz, DMSO-d6): δ 170.48, 157.08, 153.62, 153.03, 148.98, 146.80, 142.50, 126.12, 124.37, 118.52, 115.86, 115.67, 108.52, 107.60, 102.05, 72.58, 56.11, 54.60, 45.80, 41.95, 38.28, 36.28, 34.31, 26.15. HRMS (ESI) (m/z): [M + H]+ calcd for C₃₁H₃₁F₄N₅O₃, 570.2880; found, 570.2879. HPLC purity: 95.33%, retention time = 12.20 min.

### Example 7

### N-(4-(benzyloxy)phenyl)-6-methoxy-7-((1-methylpiperidin-4-yi)methoxy)quinazolin-4-amine

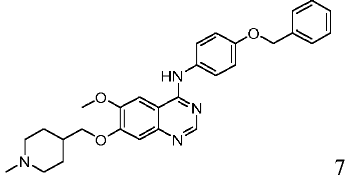

White solid (yield of 45%). ¹H NMR(400MHz, DMSO): δ 9.53 (s, 1H), 8.38 (s, 1H), 7.91 (s, 1H), 7.68 (s, 1H), 7.66 (s, 1H), 7.18 (s, 1H), 7.47 (d, J = 7.2 Hz, 1H), 7.46 (s, 1H), 7.42-7.38 (m, 2H), 7.35-7.32 (m, 1H), 7.15 (s, 1H), 7.05 (s, 1H), 7.03 (s, 1H), 5.11 (s, 2H), 4.99 (d, J = 6.0 Hz, 2H), 3.96 (s, 3H), 2.96 (d, J = 11.6 Hz, 2H), 2.32 (s, 3H), 2.23-2.17 (m, 2H), 1.84-1.81(m, 3H), 1.48-1.39 (m, 2H). 13C NMR(100 MHz, DMSO-d6) δ 158.55, 154.58, 153.29, 152.98, 148.77, 146.87, 137.20, 132.47, 128.38, 127.75, 127.64, 124.30, 114.52, 108.68, 107.75, 102.27, 72.27, 69.33, 56.33, 54.03, 44.99, 33.83, 27.53. HRMS (ESI) (m/z): [M + H]+ calcd for C₂₉H₃₂N₄O₃, 485.2553; found, 485.2556. HPLC purity: 99.64%, retention time = 12.54 min.

### Example 8

### 6-methoxy-7-((1-methylpiperidin-4-yl)methoxy)-N-(4-phenoxyphenyl)quinazolin-4-amine

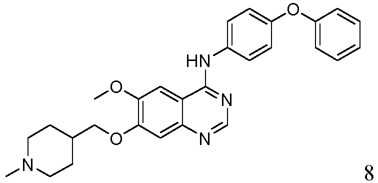

White solid (yield of 45%). ¹H NMR(400MHz, DMSO): δ 9.57 (s, 1H), 8.43 (s, 1H), 7.89 (s, 1H), 7.79 (d, J = 8.8 Hz, 2H), 7.40 (t, J = 8.4 Hz, 2H), 7.18 (s, 1H), 7.13 (t, J = 7.2 Hz, 1H), 7.07 (d, J = 8.0 Hz, 2H), 7.02 (d, J = 8.0 Hz, 2H), 4.01 (d, J = 6.0 Hz, 2H), 3.97 (s, 3H), 2.96 (d, J = 11.2 Hz, 2H), 2.32 (s, 2H), 2.18 (m, 2H), 1.90-1.88 (m, 1H), 1.83(d, J = 12.0Hz, 2H), 1.48-1.39 (m, 2H). 13C NMR(100 MHz, DMSO-d6) δ 157.82, 156.83, 153.94, 153.38, 152.48, 149.39, 147.34, 135.70, 130.47, 124.64, 123.50, 119.53, 118.45, 109.22, 108.31, 102.61, 72.84, 56.81, 54.72, 45.70, 34.42, 29.50, 28.18. HRMS (ESI) (m/z): [M + H]+ calcd for C₂₈H₃₁N₄O₃, 471.2396; found, 471.2412. HPLC purity: 97.93 %, retention time = 12.41 min.

### Example 9

### N-(3-Fluoro-4-((6-methoxy-7-((1-methylpiperidin-4-yl)methoxy)quinazolin-4-yl)amino)phenyl)-2-p henylpropionamide

Light yellow solid (yield of 35 %), melting point: 199.6-199.8°C. ¹H NMR(400MHz, DMSO-d6): δ 10.67 (s, 1H), 9.58 (s, 1H), 8.31 (s, 1H), 7.91 (s, 1H), 7.77 (d, J = 8.8 Hz, 1H), 7.46-7.42 (m, 4H), 7.34 (t, J = 7.6 Hz, 2H), 7.27-7.20 (m, 2H), 4.17-4.15 (m, 1H), 4.05 (d, J = 6.0 Hz, 2H), 3.95 (s, 3H), 3.28 (d, J = 10.4 Hz, 2H), 2.85-2.83 (m, 2H), 2.62 (s, 3H), 2.07-1.98 (m, 3H), 1.67-1.64 (m, 2H), 1.45-1.43 (d, J = 6.8 Hz, 3H). 13C NMR (100 MHz, DMSO-d6) δ 173.04, 157.91, 155.86, 153.77, 153.63, 149.29, 147.16, 142.23, 138.74, 129.00, 128.84, 127.81, 127.20, 115.17, 109.10, 108.29, 107.25, 107.00, 102.85, 72.16, 58.73, 53.22, 49.04, 46.23, 33.03, 26.42, 18.97. HRMS (ESI) (m/z): [M + H]+ calcd for C₃₁H₃₄FN₅O₃, 544.2724; found, 544.2709. HPLC purity: 98.56 %, retention time = 12.02 min.

### Example 10

### N-(3-Fluoro-4-((6-methoxy-7-((1-methylpiperidin-4-yl)methoxy)quinazolin-4-yl)amino)phenyl)-3-methyl-2-phenylbutanamide

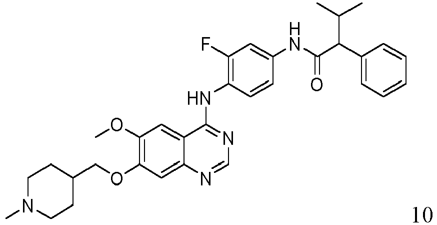

White solid (yield of 37%), melting point: 196.0-196.2°C. ¹H NMR(400MHz, DMSO-d6): δ 10.52 (s, 1H), 9.49 (s, 1H), 8.30 (s, 1H), 7.85 (s, 1H), 7.75 (dd, J1 = 2.0 Hz, J2 = 13.2 Hz, 1H), 7.45-7.38 (m, 4H), 7.33 (t, J = 7.6 Hz, 2H), 7.24 (m, 1H), 7.18 (s, 1H), 4.01 (d, J = 6.4 Hz, 2H), 3.94 (s, 3H), 3.33 (d, J = 10.4 Hz, 1H), 3.03 (d, J = 11.6 Hz, 2H), 2.37 (s, 3H), 2.34-2.24 (m, 3H), 1.91-1.84 (m, 3H), 1.50-1.45 (m, 2H), 1.04 (d, J = 6.4 Hz, 1H), 0.88 (d, J = 6.4 Hz, 1H). 13C NMR (100 MHz, DMSO-d6) δ 172.48, 157.89, 155.86, 153.93, 153.59, 149.35, 147.21, 140.04, 128.77, 128.68, 127.37, 115.19, 108.99, 108.17, 107.26, 107.00, 102.63, 72.70, 60.98, 56.65, 54.43, 45.27, 34.14, 31.47, 27.65, 21.70, 20.68. HRMS (ESI) (m/z): [M + H]+ calcd for C₃₃H₃₈FN₅O₃, 572.3037; found, 572.3047. HPLC purity: 97.51 % , retention time = 13.00 min.

### Example 11

### N-(3-fluoro-4-((6-methoxy-7-((1-methylpiperidin-4-yl)methoxy)quinazolin-4-yl)amino)phenyl)-2,2-diphenyl

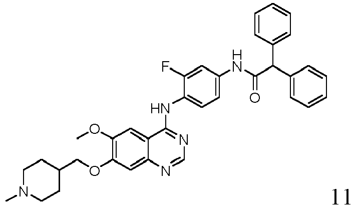

White solid (yield of 30%), melting point: 257.0-257.2°C. ¹H NMR(400MHz, DMSO-d6): δ 10.83 (s, 1H), 9.50 (s, 1H), 8.31 (s, 1H), 7.85 (s, 1H), 7.78 (d, J = 12.8 Hz, 1H), 7.47-7.34 (m, 10H), 7.29-7.26 (m, 2H), 7.18 (s, 1H), 5.27 (s, 1H), 4.01(d, J = 5.6 Hz, 2H), 3.94 (s, 3H), 3.02 (d, J = 10.8 Hz, 2H), 2.38 (s, 3H), 2.33-2.25 (m, 2H), 1.90-1.84 (m, 3H), 1.91-1.84 (m, 3H), 1.50-1.42 (m, 2H). 13C NMR (100 MHz, DMSO-d6) δ 170.64, 157.86, 155.86, 153.94, 153.60, 149.37, 147.22, 140.26, 129.04, 128.89, 127.38, 115.33, 109.00, 108.19, 107.41, 102.62, 72.69, 57.74, 56.65, 54.43, 45.25, 34.12, 27.84. HRMS (ESI) (m/z): [M + H]+ calcd for C₃₆H₃₆FN₅O₃, 606.2880; found, 606.2888. HPLC purity: 98.61 % , retention time = 13.25 min.

### Example 12

### N-(3-Fluoro-4-((6-methoxy-7-((1-methylpiperidin-4-yl)methoxy)quinazolin-4-yl)amino)phenyl)-2-( 1-oxoisoindolin-2-yl)yl)-2phenylacetamide

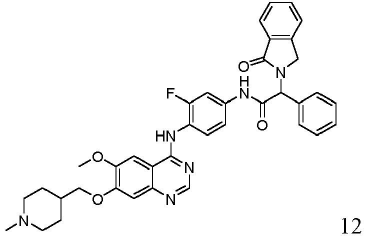

4-chloro-6-methoxy-7-((1-methylpiperidin-4-yl)methoxy)quinazoline (100 mg, 0.32 mmol) was weighted into a 100 mL eggplant-shaped bottle, and about 15mL of isopropanol was added to dissolve it. N-(4-amino-3-fluorophenyl)-2-(1-oxoisoindolin-2-yl)-2-phenylacetamide (120 mg, 0.32 mmol) was added, and 2 drops of 6 N hydrochloric acid was added and refluxed at 80°C for about 6 hours, so that a white precipitate was formed. The raw materials were consumed as detected by TLC. The reaction system was suction-filtered and the solid precipitate was washed with a small amount of isopropanol. The precipitate was dissolved in dichloromethane, and the pH was adjusted to 9 with saturated sodium bicarbonate solution. The resulting solution was extracted for several times with saturated sodium chloride / dichloromethane, and dried over anhydrous sodium sulfate. The solvent was removed *in vacuo.* The crude product was separated by silica gel column chromatography (dichloromethane / methanol = 10: 1) to obtain
N-(3-fluoro-4-((6-methoxy-7-((1-methylpiperidin-4-yl)methoxy)quinazolin-4-yl)amino)phenyl)-2-(1-oxoisoindolin-2-yl)yl)-2-phenylacetamide 90 mg, yield of 42 %. ¹H NMR (400 MHz, DMSO-d6): δ 10.80 (s, 1H), 9.45 (s, 1H), 8.32 (s, 1H), 7.82 (s, 1H), 7.78 (s, 1H), 7.75 (d, J = 4.4Hz 1H), 7.64-7.57 (m, 2H), 7.54-7.43 (m, 7H), 7.39 (d, J = 8.4Hz, 1H), 7.17 (s, 1H), 7.26 (s, 1H), 4.85 (d, J = 18Hz, 1H), 4.02-3.87 (m, 3H), 3.96 (s, 3H), 2.82 (d, J = 12.0 Hz, 2H), 2.18 (s, 3H), 2.03 (t, J = 11.2Hz, 2H), 1.81-1.78 (m, 3H), 1.38-1.35 (m, 2H). 13C NMR (100 MHz, DMSO-d6) : δ 168.34, 167.73, 157.33, 153.60, 153.06, 148.95, 146.77, 142.38, 135.18, 131.70, 131.42, 129.09, 128.54, 127.95, 123.64, 122.94, 108.45, 107.63, 102.02, 72.66, 58.54, 56.13, 54.76, 48.35, 46.05, 34.50, 28.37. HRMS (ESI) (m/z): [M + H]+ calcd for C38H37FN6O4, 661.2939; found, 661.2929. HPLC purity: 95.64 %, retention time = 12.42 min.

### Example 13

### (2-((6-methoxy-7-((1-methylpiperidin-4-yl)methoxy)quinazolin-4-yl)amino)phenyl)dimethyl phosphine oxide

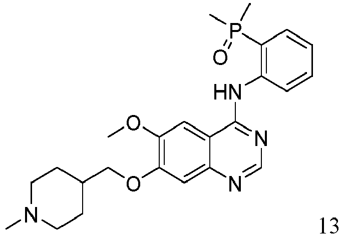

White solid (yield of 43%), melting point: 150.5-150.8°C. ¹H NMR (400 MHz, DMSO-d6): δ 12.09 (s, 1H), 9.10-9.03 (m, 1H), 8.57 (s, 1H), 7.45 (s, 1H), 7.66-7.57 (m, 2H), 7.18 (s, 1H), 7.15 (t, J = 7.2 Hz, 1H), 3.98 (d, J = 6 Hz, 2H), 3.92 (s, 3H), 2.78 (d, J = 12 Hz, 2H), 2.16 (s, 3H), 1.91-1.89 (m, 5H), 1.86 (s, 3H), 1.76-1.74 (m, 3H). 13C NMR (100 MHz, DMSO-d6): δ 155.61, 153.63, 152.62, 149.27, 146.73, 144.51, 132.43, 130.95, 120.46, 109.38, 107.94, 101.17, 72.77, 55.88, 54.80, 48.56, 46.12, 34.53, 26.41, 18.89, 18.19. HRMS (ESI) (m/z): [M + H]+ calcd for C₂₉H₃₃N₈O₃, 455.2212; found, 455.2207. HPLC purity: 97.49 %, retention time = 9.23 min.

### Example 14 (2-((7-((1-methylpiperidin-4-yl)methoxy)quinazolin-4-yl)amino)phenyl)phosphine oxide

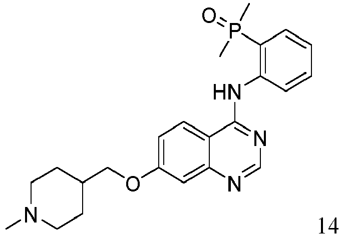

White solid (yield of 45 %), melting point: 170.7-172.4°C. ¹H NMR(400MHz, DMSO-d6): δ 12.36 (s, 1H), 9.11 (s, 1H), 8.65 (s, 1H), 8.29 (d, J = 9.2 Hz, 1H), 7.68-7.58 (m, 2H), 7.25 (dd, J1 = 2.4 Hz, J2 = 9.2 Hz, 1H), 7.23-7.15 (m, 2H), 4.02 (d, J = 6 Hz, 2H), 2.97 (d, J = 11.2 Hz, 2H), 2.33 (s, 3H), 2.20 (t, J = 10.8 Hz, 2H), 1.89 (s, 3H), 1.88 (s, 3H), 1.83 (d, J = 10.8 Hz, 3H), 1.48-1.39 (m, 2H). 13C NMR (100 MHz, DMSO-d6) : δ 162.05, 158.68, 154.77, 151.93, 144.19, 132.40, 131.04, 123.71, 122.14, 121.02, 120.95, 1118.48, 109.92, 107.79, 72.04, 54.16, 45.07, 33.95, 27.57, 18.85, 18.14. HRMS (ESI) (m/z): [M + H]+ calcd for C₂₃H₃₀N₄O₂P, 425.2106; found, 425.2110. HPLC purity: 98.02 %, retention time = 8.75 min.

### Example 15 N-(4-((6,7-Dimethoxypyridin-4-yl)amino)-3-fluorophenyl)benzamide

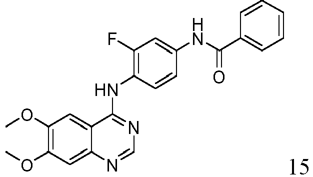

White solid (yield of 40%). ¹H NMR (400 MHz, DMSO-d6): δ 10.80 (s, 1H), 9.45 (s, 1H), 8.33 (s, 1H), 7.81 (s, 1H), 7.78 (s, 1H), 7.75(d, J = 4.4Hz 1H), 7.64-7.57 (m, 2H), 7.54-7.43 (m, 7H), 7.39 (d, J = 8.4Hz, 1H), 7.17 (s, 1H), 6.26 (s, 1H), 4.85 (d, J = 18Hz, 1H), 4.02-3.87 (m, 3H), 3.96 (s, 3H), 2.82 (d, J = 12Hz, 2H), 2.18 (s, 3H), 2.03 (t, J = 11.2Hz, 2H), 1.81-1.78 (m, 3H), 1.38-1.35 (m, 2H). 13C NMR(100 MHz, DMSO-d6): δ 165.70, 157.41, 154.21, 153.13, 148.81, 146.80, 134.65, 131.75, 128.43, 127.67, 115.87, 108.54, 107.01, 101.90, 56.05, 55.76. HRMS (ESI) (m/z): [M + H]+ calcd for C₂₉H₃₃N₈O₃, 516.2407; found, 516.2410.

### Example 16 N-(3-((6,7-Dimethoxypyridin-4-yl)amino)-4-fluorophenyl)benzamide

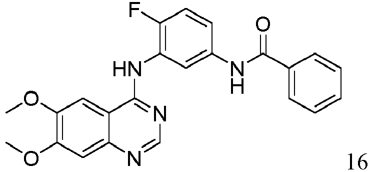

White solid, yield of 30%. ¹H NMR(400MHz, DMSO): δ 11.90(s, 1H), 10.59(s, 1H), 8.83(s, 1H), 8.47(s, 1H), 7.25(dd, J1=2.8Hz, J2=7.2Hz, 1H), 8.01 (s, 1H), 8.00 (s, 1H), 7.83-7.79 (m, 2H), 7.61 (t, J=4.8Hz, 1H), 7.56-7.52 (m, 2H), 7.47 (s, 1H), 7.40 (t, J=9.6Hz, 1H), 4.03(s, 3H), 4.00(s, J=3H). ¹³C NMR (100 MHz, DMSO-d6): δ 106.03, 157.73, 154.81, 153.49, 152.36, 149.36, 135.24, 132.11, 128.88, 128.14, 120.39, 109.10, 107.33, 102.57, 56.60, 56.30. HRMS (ESI) (m/z): [M + H]+ calcd for C₂₃H₂₀FN₄O₃, 419.1508; found, 419.1524.

### Example 17

### N-(4-((6,7-Dimethoxypyridin-4-yl)amino)-3-fluorophenyl)-2-(1-oxoisoindolin-2-yl)-2-phenylaceta mide

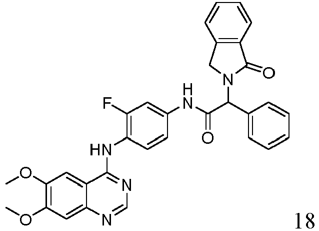

White solid, yield of 45%. ¹H NMR(400MHz, DMSO): δ 10.80 (s, 1H), 9.45 (s, 1H), 8.33 (s, 1H), 7.81 (s, 1H), 7.78 (s, 1H), 7.75 (d, J = 4.4Hz 1H), 7.64-7.57 (m, 2H), 7.54-7.43 (m, 7H), 7.39 (d, J = 8.4Hz, 1H), 7.17 (s, 1H), 6.26 (s, 1H), 4.85 (d, J = 18Hz, 1H), 4.00 (d, J = 18Hz, 1H), 3.94 (s, 3H), 3.93 (s, 3H). 13C NMR (100 MHz, DMSO-d6) : δ 168.35, 157.36, 154.22, 153.07, 148.81, 146.81, 142.39, 137.28, 135.17, 131.71, 131.42, 129.09, 128.54, 127.95, 123.65, 122.93, 108.53, 107.01, 101.90, 58.54, 56.04, 55.76, 48.57. HRMS (ESI) (m/z): [M + H]+ calcd for C₂₇H₂₅FN₄O₃,564.2012; found, 564.2045. HPLC purity: 96.86% , retention time = 14.16 min.

### Example 18

### N-(4-Fluoro-3-((6-methoxy-7-((1-methylpiperidin-4-yl)methoxy)quinazolin-4-yl)amino)phenyl)-2-( 1-oxoisoindolin-2-yl)yl)-2phenylacetamide

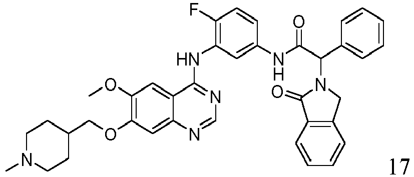

White solid (yield of 20%). ¹H NMR(400MHz, DMSO): δ 10.77 (s, 1H), 9.70 (s, 1H), 8.36 (s, 1H), 7.90 (s, 1H), 7.76 (d, J = 7.6 Hz, 1H), 7.64-7.62 (m, 2H), 7.53-7.49 (m, 2H), 7.47-7.41 (m, 4H), 7.28 (t, J = 10.0 Hz, 1H), 7.21 (s, 1H), 6.26 (s, 1H), 4.82 (d, J = 17.6 Hz, 1H), 4.03 (d, J = 17.6 Hz, 1H), 4.00 (d, J = 6.0 Hz, 2H), 3.96 (s, 3H), 3.12 (d, J = 11.6 Hz, 2H), 2.48 (s, 3H), 2.02-1.95 (m, 1H), 1.94-1.91 (m, 4H), 1.56-1.49 (m, 2H). 13C NMR(100 MHz, DMSO-d6) δ 168.64, 168.19, 157.70, 153.89, 149.45, 142.86, 135.81, 135.28, 132.17, 131.92, 129.53 129.02, 128.43, 124.12, 123.41, 119.39, 109.15, 108.11, 105.63, 103.00, 62.07, 58.96, 56.81, 56.28, 53.27, 48.89, 46.99. HRMS (ESI) (m/z): [M + H]+ calcd for C₃₈H₃₇FN₆O₄, 661.2913; found, 661.2943. HPLC purity: 95.85% , retention time = 12.42 min.

### Example 19

### (S)-N-(3-Fluoro-4-((6-methoxy-7-((1-methylpiperidin-4-yl)methoxy)quinazolin-4-yl)amino)phenyl )-2-(1-oxoisoindolin-2-yl)-2-phenylacetamide

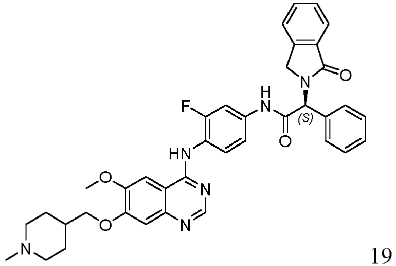

White solid (yield of 45%). ¹H NMR(400MHz, DMSO): δ 10.37 (s, 1H), 9.54 (s, 1H), 8.33 (s, 1H), 7.85 (s, 1H), 7.80 (dd, J1=2Hz, J2=5.6Hz, 1H), 7.77 (s, 1H), 7.62-7.58 (m, 2H), 7.54-7.44 (m, 7H), 7.39 (dd, J1 = 1.6 Hz, J2 = 8.8 Hz, 2H), 7.18 (s, 1H), 6.27 (s, 1H), 4.85 (d, J = 17.6 Hz, 1H), 4.02 (d, J = 5.6 Hz, 2H), 4.00 (d, J = 17.6 Hz, 2H), 3.96 (s, 3H), 2.93 (d, J = 11.2 Hz, 2H), 2.29 (s, 3H), 2.13 (t, J = 11.2 Hz, 2H). 1.82-1.80 (m, 3H), 1.48-1.38 (m, 2H). 13C NMR(100 MHz, DMSO-d6) δ 168.86, 168.23, 157.83, 155.87, 154.01, 153.57, 149.39, 147.24, 142.88, 135.86, 132.21, 129.59, 129.19, 128.45, 124.15, 123.44, 115.45, 108.98, 108.12, 107.59, 102.57, 72.89, 59.03, 58.48, 56.64, 54.79, 49.07, 48.88, 45.82, 34.50, 28.28. HRMS (ESI) (m/z): [M + H]+ calcd for C₃₈H₃₈FN₆O₄, 661.2912; found, 661.2938. HPLC purity: 97.68% , retention time = 12.48 min.

### Example 20

### (R)-N-(3-Fluoro-4-((6-methoxy-7-((1-methylpiperidin-4-yl)methoxy)quinazolin-4-yl)amino)phenyl )-2-(1-oxoisoindolin-2-yl)-2-phenylacetamide

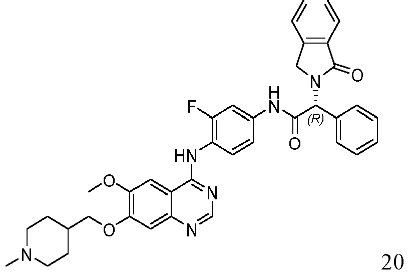

White solid (yield of 45%). ¹H NMR(400MHz, DMSO): δ 10.96 (s, 1H), 9.73 (s, 1H), 8.32 (s, 1H), 7.96 (s, 1H), 7.79-7.78 (m, 1H), 7.77 (s, 1H), 7.64-7.58 (m, 2H), 7.52 (t, J = 14 Hz, 1H), 7.49 (s, 1H), 7.47-7.43 (m, 5H), 7.39 (dd, J1 = 1.6 Hz, J2 = 8.8 Hz, 1H), 7.22 (s, 1H), 6.28 (s, 1H), 4.84 (d, J = 17.6 Hz, 1H), 4.02 (d, J = 5.6 Hz, 2H), 4.00 (d, J = 17.6 Hz, 1H), 3.96 (s, 3H), 3.22 (d, J = 11.2 Hz, 2H), 2.74 (t, J = 9.6 Hz, 2H), 2.57 (s, 3H), 1.96-1.92 (m, 3H), 1.86-1.80 (m, 2H). 13C NMR(100 MHz, DMSO-d6) δ 168.92, 168.19, 158.14, 154.06, 149.49, 142.87, 135.71, 132.20, 131.93, 129.55, 129.09, 128.99, 124.45, 123.42, 115.45 , 108.12, 107.59, 102.57, 72.90, 60.42, 59.05, 58.48, 56.98, 53.14, 49.94, 43.05, 33.00, 26.31. HRMS (ESI) (m/z): [M + H]+ calcd for C₃₈H₃₈FN₆O₄, 661.2925; found, 661.2935.

### Example 21

### 2-cyclohexyl-N-(3-fluoro-4-((6-methoxy-7-((1-methylpiperidin-4-yl)methoxy)quinazoline-4-yl)ami no)phenyl)-2-(1-oxoisoindolin-2-yl)acetamide

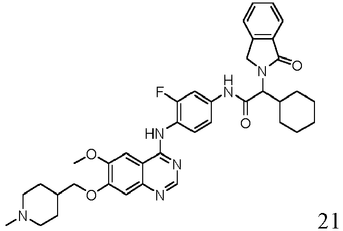

White solid (yield of 26%). ¹H NMR(400MHz, DMSO): δ 10.78 (s, 1H), 9.51 (s, 1H), 8.32 (s, 1H), 7.84 (s, 1H), 7.77 (d, J = 6.8 Hz, 1H), 7.75 (s, 1H), 7.67-7.63 (m, 2H), 7.53-7.41 (m, 3H), 7.18 (s, 1H), 4.87 (d, J = 18.4 Hz, 1H), 4.83 (s, 1H), 4.61 (d, J = 18.4 Hz, 1H), 4.00 (d, J = 6 Hz, 2H), 3.95 (s, 3H), 2.91 (d, J = 11.2 Hz, 2H), 2.26 (s, 3H), 2.11 (t, J = 10.8 Hz, 3H), 1.83-1.76 (m, 4H), 1.62-1.64 (m, 3H), 1.48-1.37 (m, 3H), 1.26-1.22 (m, 5H). 13C NMR(100 MHz, DMSO-d6) δ 169.29, 168.32, 158.23, 157.83, 155.79, 154.00, 153.56, 149.38, 147.23, 142.79, 137.68, 137.57, 129.08, 128.36, 124.04 , 123.43, 122.43, 122.31, 115.85, 108.96, 108.11, 107.72, 107.47, 102.54, 72.91, 60.24, 56.63, 54.84, 47.70, 45.90, 37.54, 34.55, 29.67, 29.58, 28.35, 26.27, 25.56, 25.41. HRMS (ESI) (m/z): [M + H]+ calcd for C₃₈H₄₃FN₆O₄, 667.3412; found, 667.3406. HPLC purity: 96.75% , retention time = 13.09 min.

### Example 22

### N-(3-Fluoro-4-((6-methoxy-7-((1-methylpiperidin-4-yl)methoxy)quinazolin-4-yl)amino)phenyl)-2-( 3-fluorophenyl)-2-(1-oxoisoindolin-2-yl)acetamide

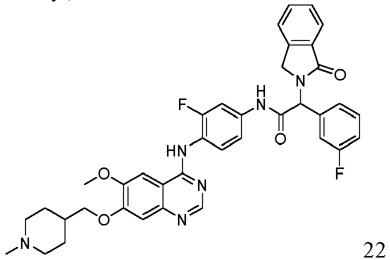

White solid (yield of 41%). ¹H NMR(400MHz, DMSO): δ 10.95 (s, 1H), 9.63 (s, 1H), 8.32 (s, 1H), 7.90 (s, 1H), 7.78 (s, 1H), 7.76 (d, J = 4.8 Hz, 1H), 7.65-7.59 (m, 2H), 7.57-7.50 (m, 2H), 7.46 (d, J = 8.8 Hz, 1H), 7.38 (d, J = 8.8 Hz, 1H), 7.31-7.26 (m, 3H), 7.22 (s, 1H), 6.26 (s, 1H), 4.82 (d, J = 17.6 Hz, 1H), 4.02 (d, J = 5.6 Hz, 2H), 4.00 (d, J = 17.6 Hz, 1H), 3.95 (s, 3H), 3.31 (d, J = 11.2 Hz, 2H), 2.84 (t, J = 9.6 Hz, 2H), 2.64 (s, 3H), 1.96-1.92 (m, 3H), 1.66-1.58 (m, 2H). 13C NMR(100 MHz, DMSO-d6) δ 168.25, 163.96, 161.52, 157.81, 155.84, 154.00, 153.57, 149.39, 147.24, 142.92, 132.28, 131.79, 129.16, 128.48, 125.18 , 124.18, 123.47, 108.98, 108.12, 102.58, 74.35, 72.87, 60.47, 58.48, 56.64, 54.76, 48.92, 45.77, 34.54, 28.25. HRMS (ESI) (m/z): [M + H]+ calcd for C₃₈H₃₇F₂N₆O₄, 679.2837; found, 679.2845. HPLC purity: 97.51% , retention time = 12.72 min.

### Example 23

### N-(3-Fluoro-4-((7-methoxy-6-((1-methylpiperidin-4-yl)methoxy)quinazolin-4-yl)amino)phenyl)-2-( 1-oxoisoindolin-2-yl)yl)-2phenylacetamide

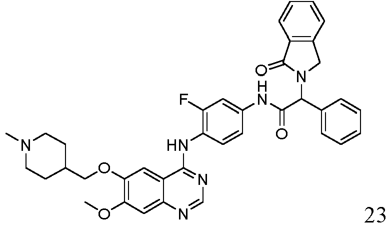

Tert-butyl ((4-((2-fluoro-4-(2-(1-oxoisoindolin-2-yl)-2-phenylacetylamino)phenyl)amino)-7-methoxyquinazolin -6-yl)oxy)methyl)piperidine-1-carboxylate (600 mg, 0.80 mmol) was weighted into a 100 mL eggplant-shaped flask, and about 12 mL of formic acid was added, and stirred at room temperature for about 30 min to dissolve it. Then a formaldehyde solution (4 mL, 47.44 mmol, 37%) was slowly add dropwise, and heated to reflux at 95°C under argon. After about 6 h, the reaction was determined to be complete by TLC. The solvent was removed by a diaphragm pump. The crude product was separated by silica gel column chromatography (dichloromethane / methanol = 10: 1) to obtain N-(3-fluoro-4-((7-methoxy-6-((1-methylpiperidine-4-yl)methoxy)quinazolin-4-yl)amino)phenyl)-2-( 1-oxoisoindolin-2-yl)yl)-2-phenylacetamide as a white solid (120 mg, yield of 22%). ¹H NMR (400MHz, DMSO): δ 10.87 (s, 1H), 9.59 (s, 1H), 8.31 (s, 1H), 7.91 (s, 1H), 7.77 (s, 1H), 7.75 (d, J = 3.2 Hz, 1H), 7.64-7.57 (m, 2H), 7.54-7.43 (m, 7H), 7.37 (d, J = 8.8 Hz, 1H), 7.19 (s, 1H), 6.25 (s, 1H), 4.24 (d, J = 17.6 Hz, 1H), 4.04-4.03 (m, 2H), 3.98 (d, J = 17.6 Hz , 1H), 3.94 (s, 3H), 3.01-2.90 (m, 2H), 2.69 (s, 3H), 2.16-2.08 (m, 1H), 2.03-1.96 (m, 3H), 1.67-1.64 (m, 2H). 13C NMR(100 MHz, DMSO-d6) δ 168.87, 168.23, 158.36, 157.88, 155.92, 154.79, 153.60, 148.52, 147.22, 142.88, 135.65, 132.21, 131.91, 129.59, 129.29, 129.05, 128.46, 124.15, 123.43, 115.48, 109.01, 107.59, 107.34, 103.29, 73.09, 59.05, 56.33, 54.03, 49.06, 44.57, 33.94, 27.52. HRMS (ESI) (m/z): [M + H]+ calcd for C₃₈H₃₇FN₆O₄, 661.2908; found, 661.2932. HPLC purity: 95.95% , retention time = 12.32 min.

### Example 24

### (3-Fluoro-4-((7-methoxy-6-((4-methylpiperazin-1-yl)methoxy)quinazolin-4-yl)amino)phenyl) -2-(-oxoisoindolin-2-yl)yl)-2-phenylacetamide

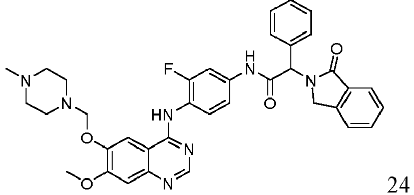

3-fluoro-4-((6-hydroxy-7-methoxyquinazolin-4-yl)amino)phenyl)-2-(1-oxoisoindolin-2-yl)-2-phenylacetamide (300 mg, 0.54 mmol) was weighted into a 100 mL eggplant-shaped bottle and ethanol was added to dissolve it. Sodium ethoxide (100 mg, 1.36 mmol), paraformaldehyde (50 mg, 1.09 mmol), N-methylpiperazine (140 mg, 1.36 mmol), 2-3 drops of concentrated HCl were added, and refluxed for about 3 hours. The reaction was determined to be complete by TLC. The solvent was removed by a water pump. The crude product was separated by silica gel column chromatography (dichloromethane / methanol = 10: 1) to obtain
N-(3-fluoro-4-((7-methoxy-6-((4-methylpiperazine-1-yl)methoxy)quinazolin-4-yl)amino)phenyl)-2-( 1-oxoisoindolin-2-yl)yl)-2-phenylacetamide as a white solid (120 mg, yield of 25%). ¹H NMR(400MHz, DMSO): δ 10.78 (s, 1H), 9.49 (s, 1H), 8.22 (s, 1H), 7.96 (s, 1H), 7.78-7.72 (m, 2H), 7.63-7.56 (m, 3H), 7.52 (d, J = 7.6 Hz, 2H), 7.48-7.43 (m, 5H), 7.33 (d, J = 8.8 Hz, 1H), 7.15 (s, 1H), 6.25 (s, 1H), 4.23 (d, J = 17.6 Hz, 1H), 3.98 (d, J = 17.6 Hz, 1H), 3.98 (s, 3H), 3.92 (s, 2H), 2.89 (s, 2H), 2.73 (s, 2H), 2.67-2.58 (m, 4H), 2.15 (s, 3H). 13C NMR(100 MHz, DMSO-d6) δ 168.75, 168.19, 158.66, 153.08, 147.81, 146.35, 142.88, 135.77, 132.18, 131.93, 129.55, 129.03, 128.44, 124.13, 123.43, 114.77, 111.46, 107.56, 107.31, 107.08, 59.98, 56.52, 54.48, 51.67, 48.87, 45.90, 45.70, 43.14. HRMS (ESI) (m/z): [M + H]+ calcd for C37H36FN7O4, 662.2883; found, 662.2885. HPLC purity: 98.81% , retention time = 12.00 min.

### Example 25

### (3-Fluoro-4-((7-methoxy-6-(3-morpholinopropoxy)quinazolin-4-yl)amino)phenyl)-2-(1-oxoisoindol in-2-yl)-2-henlacetamide

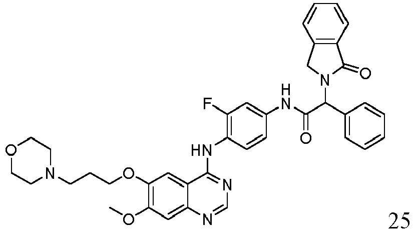

White solid (yield of 25%). ¹H NMR(400MHz, DMSO): δ 10.87 (s, 1H), 9.55 (s, 1H), 8.33 (s, 1H), 7.85 (s, 1H), 7.77 (d, J = 8.4 Hz, 2H), 7.64-7.58 (m, 2H), 7.54-7.44 (m, 7H), 7.38 (d, J = 8.8 Hz, 1H), 7.19 (s, 1H), 6.27 (s, 1H), 4.84 (d, J = 18.0 Hz, 1H), 4.17 (t, J = 6.4 Hz, 2H), 4.00 (d, J = 18.0 Hz, 1H), 3.94 (s, 3H), 3.79-3.74 (m, 4H), 2.47-2.44 (m, 2H), 2.42-2.41 (m, 4H), 2.02-1.97 (m, 2H). 13C NMR(100 MHz, DMSO-d6) δ 168.32, 168.03, 157.16, 156.97, 154.62, 152.71, 148.42, 142.36, 136.46, 134.68, 131.45, 131.39, 128.70, 128.67, 128.37, 127.59, 127.27, 123.06, 122.76, 114.96, 108.58, 107.31, 107.06, 106.86, 101.59, 66.86, 66.98, 59.25, 55.36, 54.83, 53.12, 48.24, 25.48. HRMS (ESI) (m/z): [M + H]+ calcd for C₃₈H₃₇FN₆O₅, 677.2908; found, 677.2912. HPLC purity: 95.91 % , retention time = 12.25 min.

### Example 26

### (4-((6,7-bis(2-methoxyethoxy)quinazolin-4-yl)amino)-3-fluorophenyl)-2-(1-oxoisoindolin-2-yl)-2-p henylacetamide

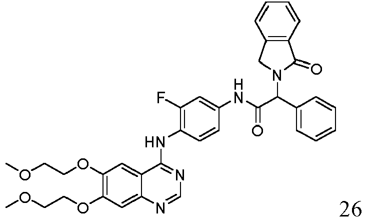

White solid (yield of 22%). ¹H NMR(400MHz, DMSO): δ 10.83 (s, 1H), 9.47 (s, 1H), 8.33 (s, 1H), 7.85 (s, 1H), 7.77 (d, J = 8.0 Hz, 2H), 7.62-7.58 (m, 2H), 7.54-7.44 (m, 7H), 7.38 (dd, J1 = 1.6 Hz, J2 = 8.8 Hz, 3H), 7.22 (s, 1H), 6.26 (s, 1H), 4.84 (d, J = 18.4 Hz, 1H), 4.27 (m, 4H), 4.99 (d, J = 18.4 Hz, 1H), 3.79-3.74 (m, 4H), 3.37 (s, 3H), 3.36 (s, 3H). 13C NMR(100 MHz, DMSO-d6) δ 168.86, 168.24, 157.82, 155.83, 154.02, 153.63, 148.44, 147.18, 142.88, 135.64, 132.22, 131.90, 129.00, 129.05, 128.46 , 124.16, 123.44, 115.43, 109.04, 108.45, 103.58, 70.52, 68.63, 68.43, 59.03, 58.84, 58.80, 48.87. HRMS (ESI) (m/z): [M + H]+ calcd for C₃₆H₃₄FN₅O₆, 652.2527; found, 642.2567. HPLC purity: 97.77% , retention time = 14.36 min.

### Example 27

### (4-((2-amino-6,7-dimethoxyquinazolin-4-yl)amino)-3-fluorophenyl)-2-(1-oxoisoindoline-2-yl)-2-phenylacetamide

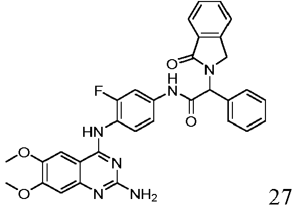

4-chloro-6,7-dimethoxy-2-amine (100 mg, 0.41 mmol) was weighted into a 100 mL eggplant-shaped bottle, about 15 mL of isopropanol was added to dissolve it. N-(4-amino-3-fluorophenyl)-2-(1-oxoisoindolin-2-yl)-2-phenylacetamide (190 mg, 0.50 mmol) was added, and 2 drops of 6 N hydrochloric acid was added and refluxed at 80°C for about 6 hours. A white precipitate was produced. The raw materials were consumed as detected by TLC. The reaction system was suction-filtered and the solid precipitate was washed with a small amount of isopropanol. The precipitate was dissolved in dichloromethane, and the pH was adjusted to 9 with saturated sodium bicarbonate solution. The resulting solution was extracted for several times with saturated sodium chloride / dichloromethane, and dried over anhydrous sodium sulfate, and the solvent was removed *in vacuo.* The crude product was separated by silica gel column chromatography (dichloromethane / methanol = 10: 1) to obtain
(4-((2-amino-6,7-dimethoxyquinazolin-4-yl)amino)-3-fluorophenyl)-2-(l-oxoisoindolin-2-yl)-2-phen ylacetamide (90 mg, yield of 37%). ¹H NMR(400MHz, DMSO): *δ* 10.66 (s, 1H), 8.18 (t, *J =* 8.8 Hz, 1H), 7.79 (s, 1H), 7.76 (d, *J=* 8.0 Hz, 1H), 7.70 (d, *J=* 12.8 Hz, 1H), 7.62-7.56 (m, 2H), 7.52-7.44 (m, 5H), 7.27-7.25 (m, 3H), 6.80 (s, 1H), 6.24 (s, 1H), 4.85 (d, *J=* 17.6 Hz, 1H), 4.97 (d, *J=* 17.6 Hz, 1H), 3.84 (s, 3H), 3.82 (s, 3H), 3.38 (s, 2H). ¹³C NMR(100 MHz, DMSO-d₆) *δ* 168.70, 168.20, 162.05, 155.24, 146.58, 142.88, 135.72, 132.19, 131.93, 129.55, 129.05, 128.45, 124.15, 123.41, 115.47, 107.34, 107.09, 104.74, 103.88, 107.08, 59.03, 56.53, 56.22, 48.91. HRMS (ESI) (*m*/*z*)*:* [M + H]⁺ calcd for C₃₂H₂₇FN₆O₄, 579.2134; found, 579.2155. HPLC purity: 98.12% , retention time = 14.71 min.

### Example 28

### 2-(2,5-Difluorophenyl)-N-(3-fluoro-4-((6-methoxy-7-((1-methylpiperidin-4-yl)methoxy)quinazolin-4-yl)amino)phenyl)-2-(1-oxoisoindolin-2-yl)acetamide

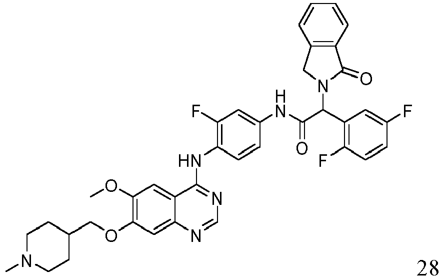

White solid (yield of 38%), melting point: 202.2-202.4°C. ¹H NMR (400 MHz, DMSO-*d*₆): δ 10.85 (s, 1H), 9.53 (s, 1H), 8.33 (s, 1H), 7.85 (s, 1H), 7.79-7.73 m, 2H), 7.66-7.29 (m, 9H), 7.18 (s, 1H), 6.42 (s, 1H), 4.82 (d, *J=* 17.6 Hz, 1H), 4.14 (d, *J=* 17.6 Hz, 1H), 4.01 (d, *J=* 6.0 Hz, 2H), 3.95 (s, 3H), 2.96 (d, *J=* 10.4 Hz, 2H), 2.32 (s, 3H), 2.19 (t, *J=* 11.2 Hz, 2H), 1.85-1.75 (m, 3H), 1.48-1.36 (m, 2H). ¹³C NMR (100 MHz, DMSO-d₆): δ 168.00, 167.40, 157.82, 155.92, 154.01, 153.57, 149.40, 147.25, 142.82, 132.38, 131.63, 129.14, 128.53, 124.23, 123.53, 115.73, 108.99, 108.15, 102.59, 72.84, 56.65, 56.68, 53.35, 48.77, 45.64, 34.38, 28.14. HRMS (ESI) (*m*/*z*)*:* [M + H]⁺calcd for C₃₈H₃₅F₃N₆O₄, 697.2750; found, 679.2759. HPLC purity: 95.36 %, retention time = 10.78 min.

### Biological evaluation method:

Tyrosine kinase:
   EGFR(WT)
   EGFR^{T790M/L858R}(LR/TM)
   EGFR^{T790M/L858R/C797S}(LR/TM/CS)
ELISA kinase activity detection

Enzyme-Linked Immunosorbent Assay (ELISA) was used to detect the ability of a kinase to phosphorylate a substrate and calculate the inhibitory effect of a compound on the kinase activity. The used kinase was EGFR L858R/T790M/C797S (purchased from BPS Bioscience).

The main steps of ELISA are listed as follows: the substrate Poly(Glu, Tyr) 4:1 was diluted with potassium ion-free PBS to 2.5 µg/well, incubated at 37°C for 12-16 h to coat the ELISA plate for use. An ATP solution (final concentration of 5 µM) diluted with a reaction buffer (50 mM HEPES pH 7.4, 20 mM MgCl₂, 0.1 mM MnCl₂, 0.2 mM Na₃VO₄, 1 mM DTT) was added to each well. A compound or solvent control was added, and then a kinase was added to start the reaction at 37°C in a shaker for 1 h. The plate was washed for three times with T-PBS, and 100 µL of antibody PY99 (diluted in T-PBS containing 5 mg/mL BSA, 1: 500) was added, and incubated in a shaker at 37°C for 0.5 h. After washing the plate with T-PBS, 100 µL of horseradish peroxidase-labeled goat anti-mouse IgG (diluted in T-PBS containing 5 mg/mL BSA, 1: 2000) was added, and incubated in a shaker at 37°C for 0.5 h. After washing the plate again, a developing liquid containing 0.03% H₂O₂, 2 mg/mL OPD (0.1 mol/L, in citrate buffer solution pH 5.4) was added at 100 µL/well, and incubated for 1-10 min at 25°C in darkness. 50 µL/well of 2 M H₂SO₄ was added to quench the reaction, and read with a wavelength-tunable microplate reader (SpectraMax Plus384, Molecular Devices) at a wavelength of 490 nm. The IC50 value was obtained from the inhibition curve.

**The results of enzyme activity test are listed as follows:**

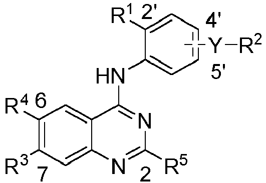

| | **R1** | **Y-R2** | **R3** | **R4** | **R5** | **EGFR kinase IC₅₀(nM)** | | |
|---|---|---|---|---|---|---|---|---|
| | | | | | | WT | LR/TM | LR/TM/CS |
| **1** | -F | 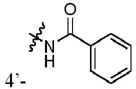 | 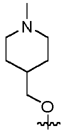 | -OMe | -H | 59.9 ± 1.8 | 6631.5 ± 657.5 | >10000 |
| **2** | -F | 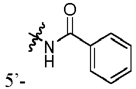 | 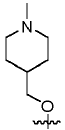 | -OMe | -H | 53.7 ± 138 | >10000 | >10000 |
| **3** | -F | 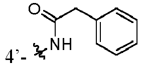 | 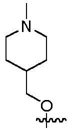 | -OMe | -H | 3.7±1.6 | 7555.2 ± 1200.0 | >10000 |
| **4** | -F | 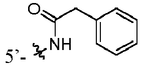 | 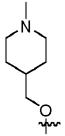 | -OMe | -H | 1346.3 ± 312.4 | >10000 | >10000 |
| **5** | -F | 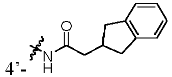 | 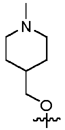 | -OMe | -H | 68.3 ± 13.0 | 1381.4 ± 234.6 | >10000 |
| **6** | -F | 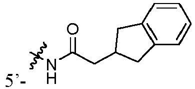 | 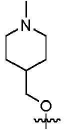 | -OMe | -H | 1095.0 ± 180.3 | 3033.4 ± 688.9 | >10000 |
| **7** | -H | 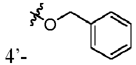 | 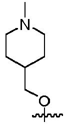 | -OMe | -H | 2.6 ± 1.3 | 4628.2 ± 825.12 | 5574.7 ± 1002.0 |
| **8** | -H | 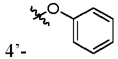 | 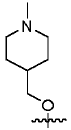 | -OMe | -H | 0.9 ± 0.2 | 191.7 ± 53.4 | 42.7 ± 5.4 |
| **9** | -F | 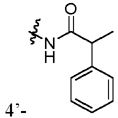 | 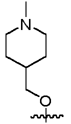 | -OMe | -H | 7.7 ± 1.8 | 2555.2 ± 800.0 | 4808.5 ± 2924.2 |
| **10** | -F | 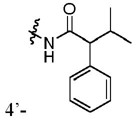 | 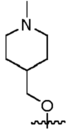 | -OMe | -H | 23.1 ± 8 | 2032.2 ± 755.3 | 3186.0 ± 2502.1 |
| **11** | -F | 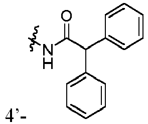 | 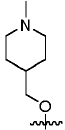 | -OMe | -H | 5.6 ± 3.3 | 1001.2 ± 435.0 | 1295.9 ± 308.2 |
| **12** | -F | 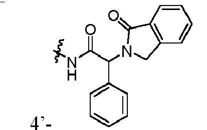 | 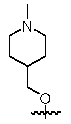 | -OMe | -H | 1.3 ± 0.3 | 3.2±0.8 | 14.5±3.3 |
| **13** | 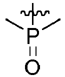 | 4'-H | 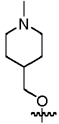 | -OMe | -H | 7341.1 ± 2515.4 | >10000 | >10000 |
| 14 | 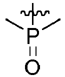 | 4'-H | 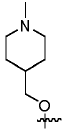 | -H | -H | 2709.8 ± 80.2 | >10000 | 9512.3 ± 526.2 |
| **15** | -F | 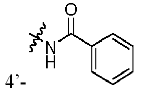 | -OMe | -OMe | -H | 113.5± 80.2 | >10000 | >10000 |
| **16** | -F | 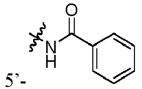 | -OMe | -OMe | -H | 137.4± 80.2 | >10000 | >10000 |
| **17** | -F | 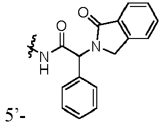 | 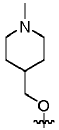 | -OMe | -H | 16.1±4.8 | 8.5±0.7 | 37.1±18.8 |
| **18** | -F | 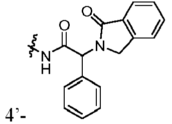 | -OMe | -OMe | -H | 1.3±0.6 | 3.2±1.3 | 14.5±5.2 |
| **19** | -F | 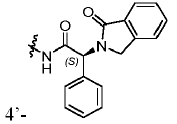 | 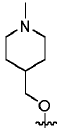 | -OMe | -H | 4.6 ± 2.8 | 8.6±2.1 | 7.9±2.0 |
| **20** | -F | 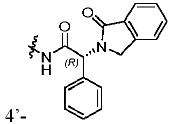 | 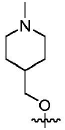 | -OMe | -H | 3.5 ± 1.0 | 6.4±1.6 | 19.2±3.2 |
| **21** | -F | 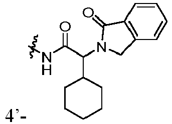 | 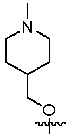 | -OMe | -H | 27.1± 12.0 | 179.6 ± 94.9 | 361.3 ± 123.4 |
| **22** | -F | 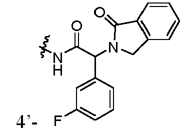 | 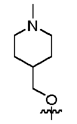 | -OMe | -H | 2.0 ± 0.5 | 6.7 ± 2.2 | 13.4 ± 2.7 |
| **23** | -F | 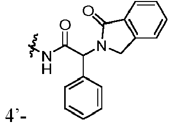 | -OMe | 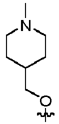 | -H | 0.8 ± 0.3 | 2.4 ± 0.5 | 18.0±8.6 |
| **24** | -F | 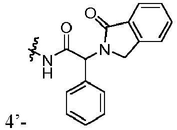 | -OMe | 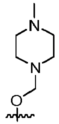 | -H | 1.0±0.2 | 4.8±0.7 | 19.1±4.6 |
| **25** | -F | 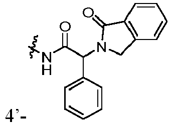 | -OMe | 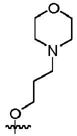 | -H | 1.6 ± 0.3 | 7.3 ± 1.3 | 97.5±12.9 |
| **26** | -F | 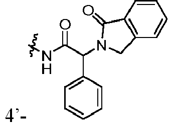 | 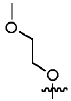 | 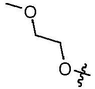 | -H | 0.7±0.1 | 1.5±0.2 | 8.5±2.6 |
| **27** | -F | 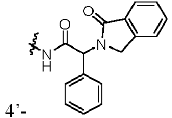 | -OMe | -OMe | -NH₂ | 388.3 ± 55.3 | >1000 | >1000 |
| **28** | -F | 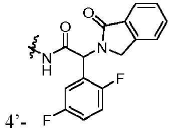 | 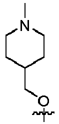 | -OMe | -H | 2.5 ± 0.8 | 35.3 ± 12.1 | 27.5±17.7 |

| | | | | | | | | |
|---|---|---|---|---|---|---|---|---|
| *^{a}* The test on kinase activity is performed by using ELISA-based EGFR-TK assay. The data are the average of at least two independent determinations and are expressed as the mean ± SD (standard deviation). *^{b}* double mutant (EGFR^{LSSSR/T790M}). *^{c}* triple mutant (EGFR^{L858R/T790M/C797S}). | | | | | | | | |

All documents mentioned in the present invention are cited as references in this application, as if each document is individually cited as a reference. In addition, it should be understood that after reading the above teachings of the present invention, a skilled person can make various changes or modifications to the present invention, and these equivalent forms also fall within the scope defined by the appended claims of the present application.

## Claims

**1.** A compound of Formula I or a stereoisomer or optical isomer, or pharmaceutically acceptable salt thereof: 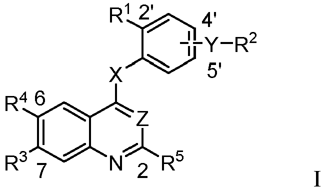 Wherein,
X is a CH₂, NH, O or S;
Z is N or a CH;
Y is absent, -O-, -NHCO-, -CONH-, -NHSO-, -SONH-, -NHCONH- or -NHSONH-;
R1 is a hydrogen, halogen (fluorine, chlorine, bromine, iodine), C1-4 alkyl, halogenated C1-4 alkyl, C1-4 alkoxy, halogenated C1-4 alkoxy, C1-4 alkylthio group, (C1-4 alkyl) (C1-4 alkyl)P(=O)-, nitro or amino;
R2 is selected from the following group: a hydrogen, substituted or unsubstituted phenyl, substituted or unsubstituted phenyl C1-4 alkyl, substituted or unsubstituted benzo C4-7 cycloalkyl, substituted or unsubstituted C4 -7 cycloalkyl C1-4 alkyl, 5 or 6-membered heterocyclic ring containing N or O, and the "substituted" means that one or more hydrogen atoms in the above group are replaced by a group selected from the following group: a halogen, C1-4 alkyl, phenyl, 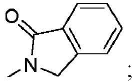
R3 and R4 are each independently selected from the group consisting of a hydrogen, substituted or unsubstituted C1-6 alkyl, substituted or unsubstituted C1-6 alkoxy, substituted or unsubstituted piperazinyl C1-6 alkoxy, substituted or unsubstituted piperidinyl C1-6 alkoxy, substituted or unsubstituted morpholinyl C1-6 alkoxy, or R3 and R4 form -O-C1-6 alkyl-O-; and the "substituted" means that one or more hydrogen atoms in the above-mentioned groups are substituted by a group selected from the group consisting of a halogen, C1-4 alkyl, C1-4 alkoxy, and phenyl;
R5 is a hydrogen, halogen, hydroxyl or amino.

**2.** The compound of claim 1 or a stereoisomer or optical isomer, or pharmaceutically acceptable salt thereof, wherein R2 is selected from the following group:

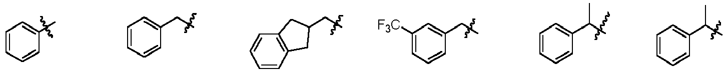

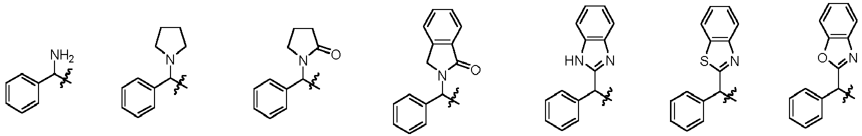

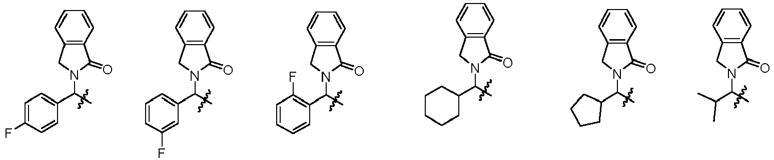

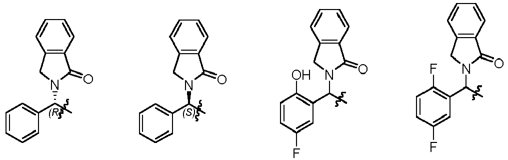

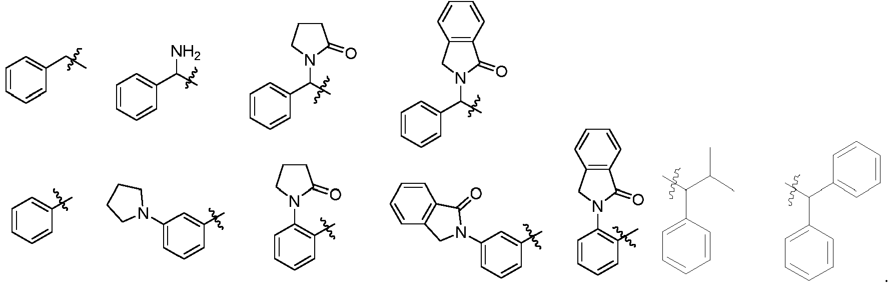

**3.** The compound of claim 1 or a stereoisomer or optical isomer, or pharmaceutically acceptable salt thereof, wherein the compound is represented by Formula II: 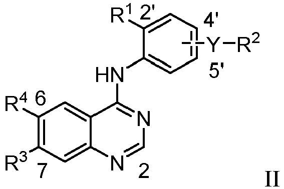 wherein R1, R2, R3, R4 and Y are as defined in claim 1.

**4.** The compound of claim 1 or a stereoisomer or optical isomer, or pharmaceutically acceptable salt thereof, wherein the compound is represented by Formula III: 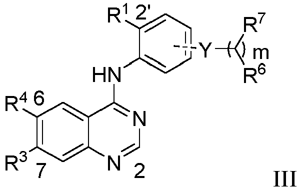
Wherein R3, R4, R1 and Y are as described in claim 1;
R6 and R7 are each independently selected from the following group: 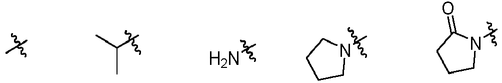 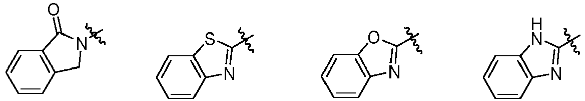 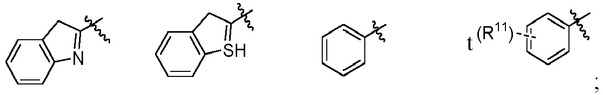
wherein R11 is selected from the group consisting of a hydrogen, halogen, and hydroxyl;
m is 0, 1 or 2;
t is 0, 1, or 2.

**6.** The compound of claim 4 or a stereoisomer or optical isomer, or pharmaceutically acceptable salt thereof, wherein m = 1.

**7.** The compound of claim 4 or a stereoisomer or optical isomer, or pharmaceutically acceptable salt thereof, wherein one of R6 and R7 is selected from the following group: 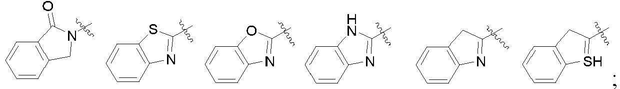 and the other is selected from the following group: 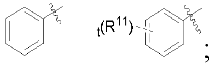 wherein n and R11 are as defined above.

**8.** The compound of any one of claims 1-7 or a stereoisomer or optical isomer, or pharmaceutically acceptable salt thereof, wherein R3 and R4 are each independently selected from the following: 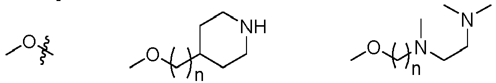 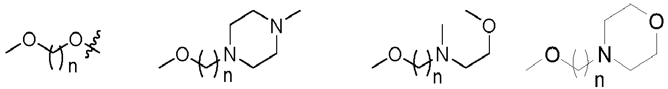 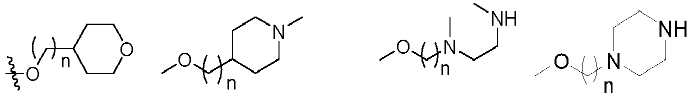 n is 1, 2 or 3.

**9.** The compound of claim 1 or a stereoisomer or optical isomer, or pharmaceutically acceptable salt thereof, wherein the compound is represented by Formula IV: 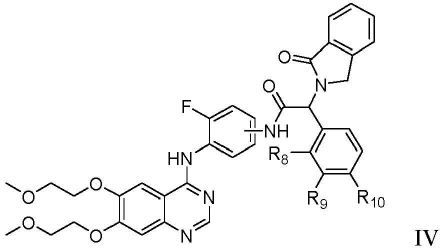 wherein R8, R9, and R10 are each independently selected from the following group: a hydrogen, halogen, and hydroxyl.

**10.** The compound of claim 1 or a stereoisomer or optical isomer, or pharmaceutically acceptable salt thereof, wherein the compound is selected from the following group:

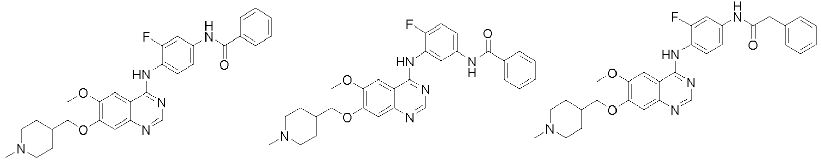

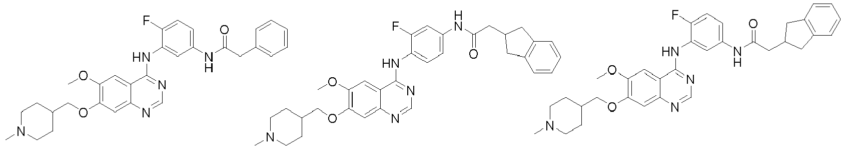

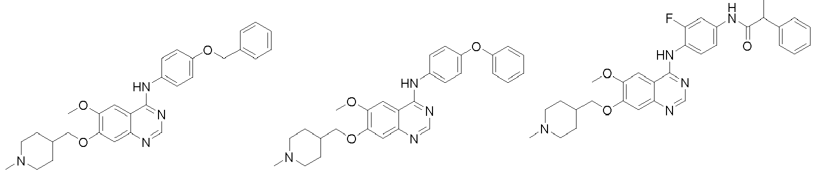

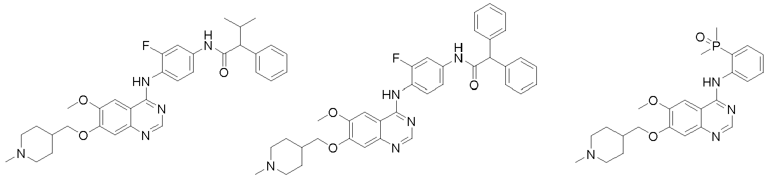

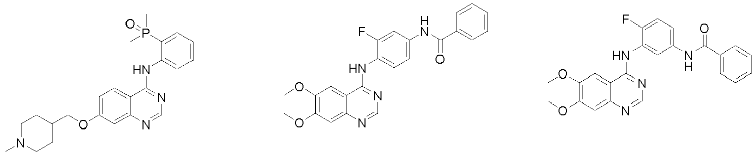

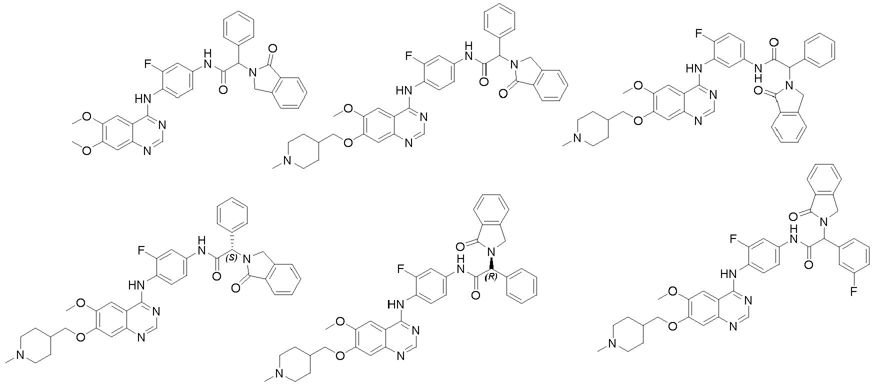

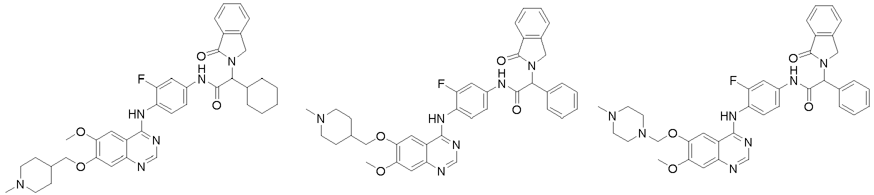

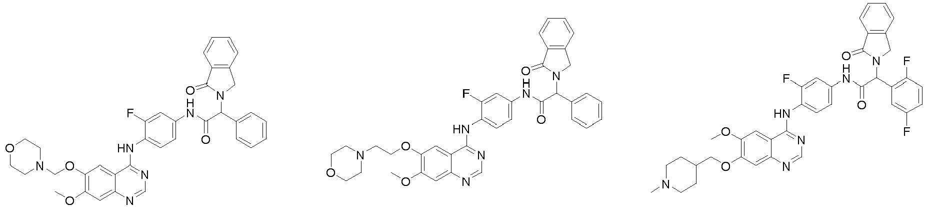

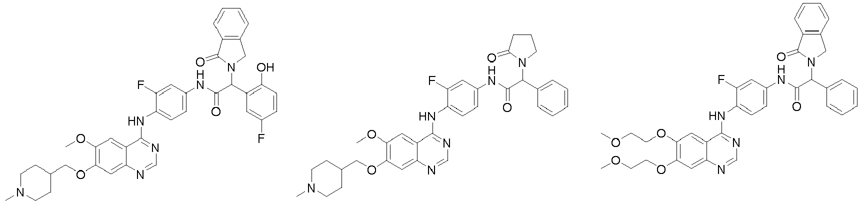

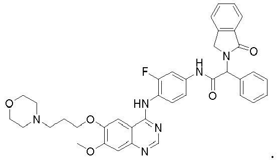

**11.** A pharmaceutical composition, comprising the compound of any one of claims 1-10 or a stereoisomer or optical isomer, or pharmaceutically acceptable salt thereof, and a pharmaceuitically acceptable carrier or excipient.

**12.** Use of the compound of any one of claims 1-10 or a stereoisomer or optical isomer, or pharmaceutically acceptable salt thereof for preparing a medicament for treating or preventing EGFR-mediated diseases or inhibiting EGFR.

**13.** The use of claim 12, wherein the EGFR-mediated disease is cancer.
